(19) Europäisches Patentamt
European Patent Office
Office européen des brevets

(11) **EP 3 103 409 B1**

(12) **EUROPÄISCHE PATENTSCHRIFT**

(45) Veröffentlichungstag und Bekanntmachung des Hinweises auf die Patenterteilung:
**18.04.2018 Patentblatt 2018/16**

(51) Int Cl.:
*A61B 34/30* (2016.01)  *A61B 34/00* (2016.01)
*A61B 90/13* (2016.01)

(21) Anmeldenummer: **16173220.1**

(22) Anmeldetag: **07.06.2016**

(54) **VORRICHTUNG ZUR ROBOTERGESTÜTZTEN CHIRURGIE UND VERFAHREN ZUM POSITIONIEREN DER VORRICHTUNG**

DEVICE FOR ROBOT-ASSISTED SURGERY AND METHOD FOR POSITIONING THE DEVICE

DISPOSITIF DE CHIRURGIE ASSISTÉ PAR UN ROBOT ET PROCÉDÉ DE POSITIONNEMENT DU DISPOSITIF

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **12.06.2015 DE 102015109368**

(43) Veröffentlichungstag der Anmeldung:
**14.12.2016 Patentblatt 2016/50**

(73) Patentinhaber: **avateramedical GmbH**
**07745 Jena (DE)**

(72) Erfinder: **Seeber, Marcel**
**07745 Jena (DE)**

(74) Vertreter: **Schaumburg und Partner Patentanwälte mbB**
**Postfach 86 07 48**
**81634 München (DE)**

(56) Entgegenhaltungen:
**US-A1- 2010 076 305      US-A1- 2011 152 871**
**US-A1- 2014 092 587      US-A1- 2014 171 957**

EP 3 103 409 B1

**Beschreibung**

Die Erfindung betrifft eine Vorrichtung zur robotergestützten Chirurgie sowie ein Verfahren zum Positionieren der Vorrichtung zur robotergestützten Chirurgie. Die Vorrichtung hat eine Instrumenteneinheit, die ein chirurgisches Instrument mit einem Instrumentenschaft umfasst. Das proximale Ende des Instrumentenschafts ist durch eine Körperöffnung eines Patienten zu einem Zielgebiet führbar. Die Instrumenteneinheit ist mit einem Manipulatorarm der Vorrichtung verbindbar.

In der minimalinvasiven Chirurgie werden zunehmend sogenannte Telemanipulatorsysteme, die auch als Roboterassistenzsysteme oder allgemein als Vorrichtung zur robotergestützten Chirurgie bezeichnet werden, eingesetzt. Mit Hilfe einer Vorrichtung zur robotergestützten Chirurgie werden chirurgische Instrumente aufgrund von Bedieneingaben in ihrer Lage und Orientierung gesteuert. Die chirurgischen Instrumente werden ferner mechanisch, elektrisch und/oder optisch an das Telemanipulatorsystem angekoppelt, um eine aktive Positionierung und Ausrichtung des chirurgischen Instruments sowie eine gewünschte Betätigung eines chirurgischen Instruments realisieren zu können. Hierzu haben die chirurgischen Instrumente, die neben Instrumenten mit Endeffektoren auch Endoskope und zu bedienende medizinische Geräte umfassen, eine Koppelschnittstelle, die als Koppeleinheit ausgebildet sein kann und auch als Sterileinheit bezeichnet wird. Die Vorrichtung zur robotergestützten Chirurgie hat ferner mindestens einen Manipulatorarm, an dessen proximalen Ende die Koppeleinheit vorgesehen ist, mit der die Sterileinheit verbindbar ist, um die mechanische, elektrische und/oder optische Kopplung zwischen dem Manipulatorarm und dem chirurgischen Instrument zu ermöglichen.

Es sind Vorrichtungen bekannt, bei denen die Manipulatorarme und die Koppeleinheiten der Manipulatorarme nicht steril und die chirurgischen Instrumente steril sind. Das sterile Operationsfeld wird von den nicht sterilen Elementen des Telemanipulatorsystems mit Hilfe einer sterilen Abdeckung geschützt. Diese sterile Abdeckung kann eine Sterilschleuse umfassen, die zwischen der Koppeleinheit des Manipulatorarms und der Sterileinheit eines chirurgischen Instruments vorgesehen ist. Eine solche Sterilschleuse ermöglicht das sterile Abdecken der nicht sterilen Koppelelemente der Koppeleinheit des Manipulatorarms nach dem Trennen der Sterileinheit der Instrumenteneinheit von dem Manipulatorarm. Eine solche Anordnung mit einer Sterilschleuse ist beispielsweise aus den Patentanmeldungen DE 10 2014 117 407 A1 und DE 10 2014 117 408 A1 bekannt.

Aus dem Dokument US 2011/0152871 A1 ist ein chirurgisches Führungssystem bekannt, das einen aktiven Roboterarm nutzt. Das Führungssystem umfasst einen Winkelanzeiger. Ein Chirurg soll ein handbetätigtes Werkzeug parallel zum Winkelanzeiger ausrichten.

Aus dem Dokument US 2014/0092587 A1 ist ein mehrfarbiger Laserpointer bekannt, bei dem der Laserpointer mit Hilfe eines Schalters ein- und ausgeschaltet werden kann und bei dem mit Hilfe eines weiteren Schalters die Farbe geändert werden kann.

Aus dem Dokument US 2010/0076305 A1 ist ein Verfahren zum Ausrichten einer Punktionsnadel bekannt. Ein Winkel zwischen einer Eintrittsstelle der Nadel und einem Tumor und der Längsachse der Nadel kann zur Prüfung der Ausrichtung der Nadel ermittelt werden. Solche Nadeln werden üblicherweise nicht in Verbindung mit Vorrichtungen zur robotergestützten Chirurgie eingesetzt.

Ferner ist aus dem Dokument US 7,666,191 B1 ein Telemanipulatorsystem bekannt, bei dem die nicht sterilen Manipulatorarme mittels einer Sterilfolie abgedeckt werden. Die Koppeleinheit des Manipulatorarms umfasst vier Rotationsaktuatoren, die mit einer ersten Seite eines in der Sterilfolie integrierten Steriladapters gekoppelt werden. Dieser Steriladapter umfasst vier integrierte drehbar gelagerte Übertragungsmittel, die zwischen der Koppeleinheit des Manipulatorarms und der Sterileinheit eines chirurgischen Instruments zwischengeschaltet sind.

Aus dem Dokument DE 102 42 953 A1 ist bekannt, einen Datensatz von einem Patientenkörper mit Hilfe eines bildgebenden Verfahrens zu erzeugen und in einem Koordinatensystem darzustellen. Weiterhin werden drei nicht in einer Ebene liegende Referenzpunkte mit dem Koordinatensystem in Verbindung gebracht.

Bei der Einrichtung bekannter Vorrichtungen zur robotergestützten Chirurgie werden die Eintrittspunkte der chirurgischen Instrumente bei einem auf einem Operationstisch liegenden Patienten festgelegt und davon ausgehend die mit den Manipulatorarmen gekoppelten chirurgischen Instrumente mit den Instrumentenspitzen auf die festgelegten Eintrittsstellen ausgerichtet. Das Ausrichten der Instrumente in Bezug auf ein Zielgebiet erfolgt durch das Bedienpersonal ausgehend von dessen Erfahrungsschatz. Eine technische Überwachung oder Kontrollmöglichkeit der Ausrichtung des Manipulatorarms des chirurgischen Instruments in Bezug auf das Zielgebiet ist im Stand der Technik nicht vorgesehen.

Aufgabe der vorliegenden Erfindung ist es, eine Vorrichtung zur robotergestützten Chirurgie und ein Verfahren zum Positionieren der Vorrichtung anzugeben, bei denen eine einfache Ausrichtung der chirurgischen Instrumente einschließlich der Endoskope in Bezug auf ein vorgesehenes Zielgebiet möglich ist. Ferner ist eine Positionierhilfseinheit zur Unterstützung beim Positionieren eines Manipulatorarms einer Vorrichtung zur robotergestützten Chirurgie anzugeben.

Diese Aufgabe wird durch eine Vorrichtung mit den Merkmalen des Anspruchs 1 sowie durch ein Verfahren mit den Merkmalen des Anspruchs 12 gelöst. Vorteilhafte Weiterbildungen der Erfindung sind in den abhängigen Ansprüchen angegeben. Eine erfindungsgemäße Vorrichtung zur robotergestützten Chirurgie hat eine Instrumenteneinheit, die ein chirurgisches Instrument umfasst, dessen proximales Ende durch eine Körperöffnung eines Patienten zu einem durch Koordinaten eines Koordinatensystems der Vorrichtung festgelegten Zielgebiet führbar ist. Ferner umfasst die Vorrichtung eine Positionierhilfseinheit, die Licht als Strahlenbündel emittiert. Die Vorrichtung umfasst mindestens einen Manipulatorarm, mit dem wahlweise die Positionierhilfseinheit oder die Instrumenteneinheit verbindbar ist. Bei einer Verbindung der Positionierhilfseinheit mit dem Manipulatorarm stimmt die Lage der Mittelachse des von der Positionierhilfseinheit emittierten Strahlenbündels mit der Lage der Längsachse des Instrumentenschafts der anstatt der Positionierhilfseinheit mit dem Manipulatorarm verbundenen Instrumenteneinheit überein. Die Vorrichtung umfasst eine Steuereinheit, die bei einer Verbindung der Positionierhilfseinheit mit dem Manipulatorarm den zur Mittelachse orthogonalen Abstandsvektor zwischen der Mittelachse und dem durch die Koordinaten festgelegten Zielgebiet ermittelt und die eine erste Steuerinformation erzeugt und vorzugsweise ausgibt, wenn der Betrag des ermittelten Abstandsvektors einen ersten voreingestellten Wert hat und/oder unterschreitet.

Durch eine solche Vorrichtung zur robotergestützten Chirurgie wird erreicht, dass der Manipulatorarm mit Hilfe der Positionierhilfseinheit einfach derart positioniert werden kann, dass eine anstatt der Positionierhilfseinheit mit dem Manipulatorarm verbundene Instrumenteneinheit in einer korrekten Position in Bezug auf eine vorgesehene Eintrittsstelle im Körper eines zu operierenden Patienten als auch in Bezug auf ein durch Koordinaten $x_z$, $y_z$, $z_z$ in einem Koordinatensystem X, Y, Z der Vorrichtung festgelegten Zielgebiet hat. Die Position umfasst dabei insbesondere die Lage und die Ausrichtung der Längsachse eines Instrumentenschafts eines chirurgischen Instruments der Instrumenteneinheit.

Dadurch ist eine einfache Ausrichtung des Manipulatorarms der Vorrichtung zur robotergestützten Chirurgie vor einem operativen Eingriff am Patienten möglich. Hierdurch ist es möglich, dass die Positionierung und Ausrichtung der Manipulatorarme nicht durch einen Arzt sondern durch hierfür geeignetes Personal durchgeführt werden kann. Dem Arzt obliegt dann lediglich die Überprüfung der Position der mit Hilfe der Positionierhilfseinheit positionierten Instrumenteneinheiten. Ein chirurgisches Instrument im Sinne der Erfindung sind insbesondere Endoskope, wie Stabendoskope, oder chirurgische Instrumente mit einem Endeffektor.

Vorteilhaft ist es, wenn die Steuereinheit zumindest eine zweite Steuerinformation dann erzeugt und vorzugsweise ausgibt, wenn der Betrag des ermittelten Abstandsvektors einen zweiten voreingestellten Wert hat oder unterschreitet. Durch die zweite Steuerinformation kann somit die korrekte Ausrichtung der Lage der Längsachse des Instrumentenschafts einer Instrumenteneinheit in Bezug auf das Zielgebiet angegeben werden.

Vorzugsweise ist der zweite voreingestellte Wert Null oder ein dem Wert Null angenäherter Wert, sodass die Mittelachse des Strahlenbündels bzw. die Längsachse des chirurgischen Instruments durch das Zielgebiet verläuft. Dadurch ist eine besonders einfache Positionierung des Manipulatorarms in Bezug auf seine Position im Raum und seine Orientierung in Bezug auf die Längsachse des chirurgischen Instruments möglich.

Der ermittelte Betrag des orthogonalen Abstandsvektors ist vorzugsweise der Betrag des kürzesten orthogonalen Abstandsvektors zwischen der Mittelachse und dem Zielgebiet.

Ferner ist es vorteilhaft, wenn die Steuereinheit die erzeugte erste Steuerinformation und die erzeugte zweite Steuerinformation zur Positionierhilfseinheit überträgt. Hierdurch wird erreicht, dass die Steuerinformation in der Positionierhilfseinheit weiterverarbeitet und ausgegeben werden kann.

Weiterhin ist es vorteilhaft, wenn die Vorrichtung und/oder die Positionierhilfseinheit eine Ausgabeeinheit hat, die aufgrund der ersten Steuerinformation ein erstes akustisches und/oder ein erstes optisches Signal ausgibt und/oder die aufgrund der zweiten Steuerinformation nur ein zweites akustisches oder ein zweites optisches Signal ausgibt. Durch die optischen und/oder akustischen Signale kann eine Bedienperson einfach über die korrekte oder noch zu korrigierende Ausrichtung der mit dem Manipulatorarm verbundenen Positionierhilfseinheit informiert werden, sodass auf einfache Weise die korrekte Positionierung des Manipulatorarms vor einem chirurgischen Eingriff unterstützt wird. Das erste und/oder zweite optische Signal kann mit Hilfe des von der Positionierhilfseinheit ermittelten Strahlenbündels ausgegeben werden, insbesondere durch eine Änderung der Wellenlänge des Wellenlängenspektrums des durch das Strahlenbündel emittierten Lichts, durch die Ausbildung/Änderung der Form des Strahlenbündels und/oder durch eine Abgabe von Lichtpulsen oder die Änderung der Frequenz und/oder Dauer der Lichtpulse.

Ferner ist es vorteilhaft, wenn das erste akustische Signal ein an- und abschwellender Ton oder eine Tonfolge mit einer ersten Wiederholfrequenz ist und dass das zweite akustische Signal ein Dauerton ist. Die Tonfolge umfasst dabei vorzugsweise mehrere gleiche Töne. Die Wiederholfrequenz kann mit einer Verringerung des ermittelten Betrags des Abstandsvektors zunehmen, sodass eine Bedienperson über die Wiederholfrequenz über eine Annäherung bzw. zunehmende Entfernung zum bzw. vom Zielgebiet akustisch informiert wird.

Die Ausgabe des akustischen Signals kann an der Steuereinheit, einer Bedienkonsole oder an der Positionierhilfseinheit erfolgen.

Alternativ oder zusätzlich kann das erste optische Signal ein blinkendes Lichtsignal mit einer ersten Blinkfrequenz sein und das zweite optische Signal ein Dauerlichtsignal. Dabei können das Licht des ersten Lichtsignals und das Licht des zweiten Lichtsignals dieselbe Wellenlänge bzw. dasselbe Wellenlängenspektrum haben. Das blinkende Signal wird insbesondere durch einen gepulsten Lichtstrahl erzeugt. Das Dauerlichtsignal wird vorzugsweise mit Hilfe eines kontinuierlichen Lichtstrahls erzeugt. Die Blinkfrequenz kann mit einer Verringerung des Betrags des Abstandsvektors zum Zielgebiet zunehmen und bei einer Vergrößerung des Betrags des Abstandsvektors zum Zielgebiet abnehmen. Die Ausgabe des optischen Signals kann an der Steuereinheit, einer Bedienkonsole oder an der Positionierhilfseinheit erfolgen.

Alternativ oder zusätzlich ist es vorteilhaft, wenn die Positionierhilfseinheit zum Erzeugen des ersten optischen Signals Licht mit einer ersten Wellenlänge und zum Erzeugen des zweiten optischen Signals Licht mit einer von der ersten Wellenlänge verschiedenen zweiten Wellenlänge emittiert. Zum Erzeugen der optischen Signale wird Licht mit einer Wellenlänge im sichtbaren Bereich eingesetzt. Vorzugsweise ist das Licht der ersten Wellenlänge rotes Licht und das Licht der zweiten Wellenlänge grünes Licht. Hierdurch erfolgt eine Information der Bedienperson über die Position und Ausrichtung des Manipulatorarms, die mit Hilfe der Lichtsignale einfach intuitiv erfasst werden kann. Die Ausgabe des optischen Signals kann an der Steuereinheit, einer Bedienkonsole oder an der Positionierhilfseinheit erfolgen.

Besonders vorteilhaft ist es, wenn die Positionierhilfseinheit zum Erzeugen des ersten und/oder des zweiten optischen Signals mit einer Einheit ausgerüstet ist, die ein Strahlenbündel emittiert, durch das mit Hilfe des Strahlenbündels eine Abbildung eines Fadenkreuzes und/oder mindestens eines um die Mittelachse des Strahlenbündels konzentrisch angeordneten Kreises auf eine Projektionsfläche erfolgt. Die Projektionsfläche kann dabei die Körperoberfläche des Patienten oder ein bereits in dem Körper des Patienten eingeführter Trokar sein. Dadurch ist es einfach möglich, den Lichtstrahl auf eine vorhandene oder geplante, insbesondere eine bereits markierte, Eintrittsstelle zum Einführen des chirurgischen Instruments in den Körper des Patienten auszurichten. Das Fadenkreuz und/oder der konzentrische angeordnete Kreis lassen sich einfach auf diese gewünschte Eintrittsstelle ausrichten.

Dabei ist es vorteilhaft, wenn der Lichtstrahl zum Erzeugen des ersten und/oder zweiten optischen Signals in einem Winkel im Bereich zwischen 15° und 35° abgestrahlt wird. Hierdurch wird ein geeigneter Abbildungsbereich zum Projizieren des optischen Signals auf eine Oberfläche des Patienten oder auf einen in den Körper des Patienten eingeführten Trokars einfach ermöglicht. Besonders vorteilhaft ist es, wenn mit Hilfe des Strahlenbündels zum Erzeugen des ersten und/oder des zweiten optischen Signals eine Abbildung von mehreren um die Mittelachse des Strahlenbündels konzentrisch angeordneten Kreisen erfolgt, wobei die Kreise vorzugsweise den gleichen Winkelabstand in Bezug auf den Gesamtabstrahlwinkel haben. Vorzugseise ergibt sich der Betrag des Abstandwinkels alpha(k) zwischen den einzelnen Kreisen nach folgender Gleichung:

$$alpha(k)=alpha(g)/(2*i)$$

wobei

alpha(g) der Gesamtabstrahlwinkel und
i die Anzahl der Kreise ist.

Bei fünf Kreisen beträgt der Winkelabstand alpha(k) der Kreise untereinander 2,82° bei einem Gesamtabstrahlwinkel von 28,2°.

Ferner ist es vorteilhaft, wenn die Positionierhilfseinheit eine Energiequelle zum Versorgen einer Signalerzeugungseinheit zum Erzeugen des ersten und/oder zweiten optischen und/oder zum Erzeugen des ersten akustischen und/oder zweiten akustischen Signals hat. Die Signalerzeugungseinheit kann Bestandteil einer elektrischen Schaltung sein. Hierdurch können die zum Positionieren des Manipulatorarms mit Hilfe der Positioniereinheit erforderlichen Informationen einfach indirekt von der Positionierhilfseinheit ausgegeben werden, wodurch ein einfacher und kompakter Aufbau ermöglicht wird.

Die Energieversorgungseinheit kann eine Batterie, einen Akkumulator, ein Kondensator oder eine Anordnung zur drahtlosen Energieübertragung von einer Koppeleinheit des Manipulatorarms zur Positionierhilfseinheit haben. Die Energieversorgungseinheit kann auf Seiten der Koppeleinheit des Manipulatorarms auch eine RFID-Lese und/oder Schreibeinheit umfassen, mit deren Hilfe eine RFID-Anordnung der Positionierhilfseinheit mit Energie versorgt und eine Datenübertragung zwischen der RFID-Lese- und/oder Schreibeinheit und der Positionierhilfseinheit ermöglicht wird. Alternativ oder zusätzlich kann eine Spulenanordnung zur Energieübertragung eingesetzt werden, wie sie zur drahtlosen Energieversorgung allgemein im Stand der Technik bekannt ist.

Ferner ist es vorteilhaft, wenn die Steuersignale der Steuereinheit und/oder weitere Informationen drahtlos mit Hilfe einer drahtlosen Datenübertragung, optisch über eine optische Schnittstelle oder über eine fest verdrahtete Verbindung über elektrische Kontakte zur Positionierhilfseinheit übertragbar sind. Hierzu kann die Positionierhilfseinheit einen RFID- Transponder haben, wobei die Informationen in ein Register bzw. einen Speicher des RFID-Transponders geschrieben werden. Die erste und zweite Steuerinformation können mit Hilfe einer RFID-Lese- und/oder Schreibeinheit der Koppeleinheit des Manipulatorarms zum RFID-Transponder übertragen werden. Zum Erzeugen des ersten und/oder zweiten optischen Signals umfasst die Positionierhilfseinheit vorzugsweise eine Lichtquelle, insbesondere umfasst die Lichtquelle mindestens eine Laserlichtquelle, mindestens eine einfarbige oder mehrfarben LED-Lichtquelle, eine mindestens zwei LEDs umfassende LED-Lichtquelle, wobei die LEDs Licht in unterschiedlichen Wellenlängen emittieren, mindestens eine Glühlampe mit oder ohne Farbfilter. Hierdurch kann das Licht zum Emittieren des Strahlenbündels einfach in der Positionierhilfseinheit erzeugt werden.

Die Energie- und/oder Datenübertragung kann auch über elektrische Kontakte und/oder drahtlos und/oder über eine optische Schnittstelle bidirektional zwischen der Positionierhilfseinheit und der Koppeleinheit erfolgen.

Ferner ist es vorteilhaft, wenn sowohl die Positionierhilfseinheit als auch die Instrumenteneinheit über einen mit einer Koppeleinheit des Manipulatorarms verbundene Sterilschleuse mit dem Manipulatorarm verbindbar sind. Über diese Sterilschleuse kann eine sterile Trennung der nicht sterilen Koppeleinheit von dem sterilen Bereich erfolgen. Beim Koppeln der Sterileinheit der Instrumenteneinheit mit der Sterilschleuse werden vorzugsweise Sterilklappen der Sterilschleuse geöffnet, sodass eine direkte Verbindung zwischen Elementen der Sterileinheit und der Koppeleinheit hergestellt werden kann. Beim Verbinden der Positionierhilfseinheit mit der Sterilschleuse werden die Schleusenklappen geöffnet oder bleiben alternativ geschlossen. Nach dem Trennen der Positionierhilfseinheit von der Sterilschleuse sind die Schleusenklappen geschlossen. Vorzugsweise hat die Sterileinheit Sterilklappen, die beim Verbinden der Sterileinheit mit der Sterilschleuse geöffnet werden, sodass eine direkte Verbindung zwischen Übertragungselementen, insbesondere von mechanischen Antriebselementen, zwischen der Koppeleinheit und der Sterileinheit möglich ist. Nach dem Trennen der Sterileinheit von der Sterilschleuse werden sowohl die Schleusenklappen als auch die Sterilklappen wieder geschlossen, sodass sowohl die Übertragungselemente der Sterileinheit steril abgedeckt sind als auch die Übertragungselemente der Koppeleinheit. Über die Sterileinheit sind darüber hinaus elektrische und/oder optische Verbindungen zwischen der Koppeleinheit und der Positionierhilfseinheit herstellbar.

Ein weiterer Aspekt betrifft eine Positionierhilfseinheit zur Unterstützung beim Positionieren eines Manipulatorarms in einem Koordinatsystem einer Vorrichtung zur robotergestützten Chirurgie, wobei die Positionierhilfseinheit anstelle einer Instrumenteneinheit mit einer Koppeleinheit des Manipulatorarms verbindbar ist. Die Positionierhilfseinheit hat eine Lichtquelle, die Licht als Strahlenbündel emittiert, wobei die Lage der Mittelachse des von der Lichtquelle emittierten Strahlenbündels der mit der Koppeleinheit verbundenen Positionierhilfseinheit in der Lage der Längsachse des chirurgischen Instruments der mit der Koppeleinheit verbundenen Instrumenteneinheit übereinstimmt. Ferner hat die Positionierhilfseinheit eine elektronische Schaltung mit einer Schnittstelle zum Empfangen einer ersten Steuerinformation, die angibt, dass der Betrag eines ermittelten zur Mittelachse orthogonalen Abstandsvektors zwischen einem durch Koordinaten $x_z$, $y_z$, $z_z$ des Koordinatensystems festgelegten Zielgebiet und der Mittelachse einen ersten voreingestellten Wert hat oder unterschreitet. Mit Hilfe einer solchen Positionierhilfseinheit kann ein Manipulatorarm auf einfach Weise in Bezug auf ein festgelegtes Zielgebiet positioniert werden, d. h. in eine entsprechende Lage und Ausrichtung gebracht, werden. Die Schnittstelle der elektronischen Schaltung ist vorzugsweise eine Schnittstelle zu einer Steuereinheit der Vorrichtung, die vorzugsweise den Betrag eines zur Mittelachse orthogonalen Abstandsvektors zwischen einem durch Koordinaten $x_z$, $y_z$, $z_z$ des Koordinatensystems festgelegten Zielgebiet und der Mittelachse ermittelt und prüft, ob der ermittelte Betrag einen ersten voreingestellten Wert hat oder unterschreitet. Die Steuereinheit erzeugt abhängig vom Ergebnis der Prüfung die erste und/oder eine zweite Steuerinformation und überträgt diese über die Schnittstelle zur elektronischen Schaltung. Ferner kann die Schnittstelle der elektronischen Schaltung zum Empfangen einer zweiten Steuerinformation dienen, die angibt, dass der Betrag des Abstandsvektors zwischen den Koordinaten $x_z$, $y_z$, $z_z$ des Zielgebiets und der Mittelachse einen zweiten voreingestellten Wert hat oder unterschreitet. Vorzugsweise ist der zweite voreingestellte Wert Null.

Ein weiterer Aspekt betrifft ein Verfahren zum Positionieren eines Manipulatorarms in einem Koordinatsystem einer Vorrichtung zur robotergestützten Chirurgie, bei dem die Koordinaten $x_z$, $y_z$, $z_z$ eines Zielgebiets eines Patienten ermittelt werden. Zum Positionieren eines Manipulatorarms der Vorrichtung zur robotergestützten Chirurgie insbesondere zur Vorbereitung einer Operation an einem Patienten wird anstelle einer Instrumenteneinheit eine Positionierhilfseinheit mit einer Koppeleinheit des Manipulatorarms verbunden. Durch die Positionierhilfseinheit wird Licht als Strahlenbündel emittiert, wobei die Lage der Mittelachse des von der mit der Koppeleinheit verbundenen Positionierhilfseinheit emittierten Strahlenbündels mit der Lage der Längsachse des chirurgischen Instruments der anstelle der Positionierhilfseinheit mit dem Manipulatorarm verbundene Instrumenteneinheit übereinstimmt. Die Längsachse des chirurgischen Instrumentes ist insbesondere die Längsachse des Instrumentenschafts des chirurgischen Instruments. Bei einer Verbindung der Positionierhilfseinheit mit dem Manipulatorarm wird mit Hilfe der Steuereinheit der Betrag eines zur Mittelachse orthogonalen Abstandsvektors zwischen der Mittelachse und dem durch die Koordinaten $x_z$, $y_z$, $z_z$ festgelegten Zielgebiet ermittelt. Es wird ein erstes optisches und/oder akustisches Signal ausgegeben, wenn der ermittelte Betrag einen ersten voreingestellten Wert hat und/oder unterschreitet. Dadurch wird sichergestellt, dass die Mittelachse des Strahlenbündels bzw. nachfolgend die Längsachse des chirurgischen Instruments sowohl durch die geplante, insbesondere bereits markierte, oder vorhandene operative Körperöffnung des Patienten als auch durch das Zielgebiet verläuft. Dadurch ist eine einfache intuitive korrekte Positionierung des Manipulatorarms insbesondere zur Vorbereitung der Vorrichtung zur robotergestützten Chirurgie für einen chirurgischen Eingriff einfach und möglich. Hierfür ist kein speziell geschultes medizinisches Fachpersonal erforderlich, insbesondere kein Arzt.

Ferner ist es vorteilhaft, wenn ein zweites optisches und/oder akustisches Signal dann ausgegeben wird, wenn der ermittelte Betrag des Abstandsvektors einen zweiten voreingestellten Wert hat oder diesen unterschreitet. Vorzugsweise ist der zweite voreingestellte Wert Null.

Der Manipulatorarm wird vorzugsweise manuell so ausgerichtet, dass die Mittelachse des Strahlenbündels durch eine geplante, insbesondere markierte, oder vorhandene operative Körperöffnung des Patienten verläuft. Weiterhin wird der Manipulatorarm so ausgerichtet, dass das erste und/oder zweite optische und/oder akustische Signal ausgegeben wird.

Ferner ist es auch vorteilhaft, wenn der Manipulatorarm zunächst so ausgerichtet wird, dass die Mittelachse des Strahlenbündels durch eine geplante, insbesondere bereits markierte, oder tatsächliche operative Körperöffnung eines Patienten verläuft und das erste und/oder zweite optische und/oder akustische Signal ausgegeben wird. Hierdurch ist eine einfache weitere Ausrichtung und Positionierung des Manipulatorarms möglich, bis der Betrag des ermittelten Abstandsvektors einen zweiten Wert hat oder diesen unterschreitet.

Es ist vorteilhaft, wenn der Manipulatorarm in einem ersten Schritt so ausgerichtet wird, dass die Mittelachse des Strahlenbündels durch eine geplante oder vorhandene operative Körperöffnung des Patienten verläuft, und dass der Manipulatorarm in einem zweiten Schritt so bewegt wird, dass der mit Hilfe der Steuereinheit ermittelte Abstand zwischen der durch die geplante oder vorhandene operative Körperöffnung des Patienten verlaufenden Mittelachse und dem durch die Koordinaten festgelegten Zielgebiet den zweiten voreingestellten Abstand hat oder unterschreitet, sodass das zweite optische und/oder akustische Signal ausgegeben wird. Der Manipulatorarm wird dazu automatisch durch die Vorrichtung selbst oder manuell bewegt. Auch kann ein Teil der Ausrichtbewegung durch die Vorrichtung selbst automatisch und ein Teil der Ausrichtbewegung manuell durch eine Bedienperson erfolgen.

Zusätzlich kann in einem dritten Schritt die Positionierhilfseinheit vom Manipulatorarm getrennt werden und in einem vierten Schritt die Instrumenteneinheit mit dem Manipulatorarm verbunden werden. Dabei bleibt sowohl im dritten als auch im vierten Schritt die Position und Ausrichtung des Manipulatorarms unverändert in der im zweiten Schritt festgelegten Position und Ausrichtung.

Alternativ kann der Manipulatorarm in einem ersten Schritt so ausgerichtet werden, dass der mit Hilfe der Steuereinheit ermittelte Betrag des Abstandsvektors zwischen der Mittelachse und der Koordinaten $x_z$, $y_z$, $z_z$ des Zielgebiets den zweiten voreingestellten Abstand hat oder unterschreitet. Der Manipulatorarm wird dann in einem zweiten Schritt so ausgerichtet, dass die Mittelachse des Strahlenbündels durch die vorhandene oder geplante, insbesondere markierte, operative Körperöffnung des Patienten verläuft und dabei die Mittelachse des Strahlenbündels auf das Zielgebiet ausgerichtet bleibt. Dabei kann der Manipulatorarm automatisch durch die Vorrichtung selbst und/oder manuell durch eine Bedienperson im ersten und/oder zweiten Schritt bewegt werden.

Besonders vorteilhaft ist es, wenn in einem dritten Schritt die Positionierhilfseinheit vom Manipulatorarm getrennt wird und in einem vierten Schritt die Instrumenteneinheit mit dem Manipulatorarm verbunden wird. Die Position und Ausrichtung des Manipulatorarms bleibt während der Ausführung des dritten und des vierten Schritts unverändert in der durch den zweiten Schritt bewirkten Position, d.h. Lage und Ausrichtung.

Durch die aufgezeigten Lösungen der Aufgabe der Erfindung durch die unabhängigen und abhängigen Ansprüche ist es möglich, eine optimale Vorpositionierung der Instrumenteneinheit durch eine manuelle und oder automatische Vorpositionierung der Manipulatorarme der Vorrichtung zur robotergestützten Chirurgie zu erreichen. Erfindungsgemäß wird dazu die Positionierhilfseinheit anstelle der eigentlichen Instrumenteneinheit mit dem chirurgischen Instrument mit der Koppeleinheit des Manipulatorarms verbunden. Hierbei übernimmt die Positionierhilfseinheit mehrere Funktionen:

1. Durch Projektion des Strahlenbündels auf die Oberfläche eines Patienten insbesondere durch die Projektion eines optisch erkennbaren Musters, wie einem Fadenkreuz oder konzentrischen Kreisen oder ähnlichen, wird für den Bediener die Positionierung der geplanten oder vorhandenen Körperöffnungen zum Einführen des chirurgischen Instruments in den Körper des Patienten angezeigt. Der Lichtstrahl bzw. das Muster zeigt dabei den Punkt auf dem Körper des Patienten, an dem ein chirurgisches Instrument einer mit dem Manipulatorarm verbundene Instrumenteneinheit in den Situs eindringen würde. An dieser Stelle wird dann ein Trokar eingesetzt, durch den dann der Instrumentenschaft des chirurgischen Instruments hindurchgeführt wird.

2. Durch die Farbe des projizierten Strahlenbündels oder Musters und/oder durch ein akustisches Signal wird eine Information an eine Bedienperson ausgegeben, die angibt, ob die Orientierung, d. h. die Ausrichtung, einer durch eine Koppeleinheit des Manipulatorarms gebildete Koppelschnittstelle so erfolgt ist, dass die Verlängerung eines Instrumentenschafts eines chirurgischen Instruments der Instrumenteneinheit durch das Zielgebiet verläuft. So kann beispielsweise ein grünes Fadenkreuz die richtige Orientierung des Instrumentenschafts anzeigen, während ein rotes Fadenkreuz signalisiert, dass die Orientierung des Instrumentenschafts einer mit dem Manipulatorarm zu verbindenden Instrumenteneinheit nicht so ausgerichtet ist, dass die Verlängerung des Instrumentenschafts bzw.

dessen Längsachse nicht auf das Zielgebiet zeigt. Der Manipulatorarm kann eine Teleskopanordnung zum Bewegen der Koppeleinheit in Richtung der Teleskopachse der Teleskopanordnung haben. Die Lage der Teleskopanordnung wird beim Ausfahren und Einfahren der Teleskopanordnung so gesteuert, dass sich der Instrumentenschaft entlang seiner verlängerten Längsachsebewegt, d.h. die Lage der Längsachse im Raum bleibt konstant.

Durch die Kombination der beiden zuvor beschriebenen Funktionen erkennt der Bediener, ob die Positionierung (Lichtstrahl trifft auf die gewünschte Eintrittsstelle in den Situs) und die Orientierung entsprechende Farbe des Strahlenbündels (beispielsweise Grün und/oder akustisches Signal) gleichzeitig erfolgreich eingestellt hat. In dieser Position kann ein in den Situs eingeführter Trokar optimal mit einer hierfür optional vorgesehenen Koppelschnittstelle des Manipulatorarms verbunden werden. In jedem Fall ist in dieser Position der Manipulatorarm in einer optimalen Ausgangslage für einen geplanten operativen Eingriff.

Vorzugsweise sind die relevanten Bereiche der Anatomie des Patienten, insbesondere das Zielgebiet, durch die Koordinaten $x'_z$, $y'_z$, $z'_z$ in einem Patientenkoordinatensystem X', Y', Z' bestimmt. Der Koordinatenursprung des Patientenkoordinatensystems X', Y', Z' kann beispielsweise im Kreuzungspunkt der Medianebenen mit der dorsal liegenden Frontalebene sowie einer Transversalebene festgelegt sein. Die Koordinaten des Patientenkoordinatensystems stehen in fester bekannter Beziehung zu einem Koordinatensystem X, Y, Z der Vorrichtung zur robotergestützten Chirurgie, sodass die Koordinaten x, y, z von Elementen der Vorrichtung einfach in Koordinaten x', y', z' des Patientenkoordinatensystems X', Y', Z' umrechnen lassen und Koordinaten x', y', z' des Patientenkoordinatensystems X', Y', Z', wie z. B. die Koordinaten $x'_z$, $y'_z$, $z'_z$ des Zielgebiets, sich einfach in Koordinaten $x_z$, $y_z$, $z_z$ des Koordinatensystems X, Y, Z der Vorrichtung umrechnen lassen.

Beispielsweise können die Koordinaten $x'_z$, $y'_z$, $z'_z$ eines Zielgebiets im Patientenkoordinatensystem X', Y', Z' dadurch bestimmt werden, dass eine manuelle Vermessung des Patienten, z. B. mit Hilfe eines Maßbandes, durchgeführt wird. Dabei liefert eine zentimetergenaue Bestimmung der Koordinaten $x_z$, $y_z$, $z_z$ des Zielgebiets in den Koordinaten des Patientenkoordinatensystems X', Y', Z' und deren Transformation in Koordinaten $x_z$, $y_z$, $z_z$ des Koordinatensystems X, Y, Z der Vorrichtung eine vielfach ausreichende Genauigkeit, um das chirurgische Instrument ausreichend gut positionieren und ausrichten zu können. Alternativ kann die Vermessung des Zielgebietes auch direkt in Koordinaten $x_z$, $y_z$, $z_z$ des Koordinatensystems der Vorrichtung erfolgen.

Darüber hinaus liefern moderne Verfahren der Bildgebung, wie z. B. die Computertomographie oder die Magentresonanztomographie Daten, die eine genauere Bestimmung der Koordinaten im Patientenkoordinatensystem X', Y', Z' und davon ausgehend im Koordinatensystem der Vorrichtung ermöglichen.

Vorzugsweise hat die Positionierhilfseinheit einen Schaft, dessen Lage und Ausrichtung mit der Lage und der Ausrichtung des Instrumentenschafts des chirurgischen Instruments einer anstatt der Positionierhilfseinheit mit dem Manipulatorarm verbindbaren Instrumenteneinheit übereinstimmt. Der Schaft der Positionierhilfseinheit hat vorzugsweise eine Länge, die im eingefahrenen Zustand der Teleskopanordnung des Manipulatorarms gerade so lang ist, dass der Schaft ca. 1 cm tief in einen in den Situs eingeführten Trokar eingeführt wird. In diesem Zustand kann auch eine optional am Manipulatorarm vorhandene Trokar-Halterung mit dem Trokar verbunden werden. Im eingefahrenen Zustand der Teleskopanordnung hat das proximale Ende des Schafts einen Abstand im Bereich von 5 cm bis 30 cm, vorzugsweise im Bereich von 10 cm bis 25 cm und/oder kontaktiert mit seinem proximalen Ende ein Führungselement der optionalen Trokar-Halterung. Durch die Ausrichtung und Manipulatorarms mit Hilfe der Positionierhilfseinheit ist die Trokar-Halterung quasi automatisch richtig zum Verbinden mit dem Trokar positioniert, wenn der Schaft der Positionierhilfseinheit 1 cm tief in den Trokar eintaucht.

In Kombination mit präoperativ ermittelten Daten des Zielgebiets, insbesondere mit Hilfe einer Computertomographie oder Magentresonanztomographie, kann während eines Einricht- und Andockvorgangs mit Hilfe der Positionierhilfseinheit die Lage der Verlängerung der Mittelachse des Strahlenbündels bzw. der Längsachse des Instrumentenschafts nach dem Austausch der Positionierhilfseinheit gegen die Instrumenteneinheit zum Zielgebiet auf einem zusätzlichen im Blickfeld des Bedieners angebrachten Monitor dargestellt werden. Vorzugsweise wird hierzu eine zur Längsachse des Instrumentenschaftes orthogonal liegende Schnittebene durch den CT und/oder MRT-Datensatz des Patienten dargestellt. Die Schnittebene durch den CT- und/oder MRT-Datensatz geht durch das Ziel-OP-Gebiet hindurch. Damit erhält der Bediener neben dem projizierten Lichtstrahl an der vorhandenen oder geplanten operative Körperöffnung und der zusätzlichen akustischen und optischen Information über den Abstand der Mittelachse des Strahlenbündels bzw. der Längsachse des chirurgischen Instruments zum Zielgebiet eine weitere Entscheidungshilfe, um eine optimale Einrichtung der Manipulatorarme der Vorrichtung zur robotergestützten Chirurgie für den geplanten chirurgischen Eingriff vornehmen zu können. Die Vorrichtung zur robotergestützten Chirurgie hat vorzugsweise mehrere, insbesondere vier oder fünf, Manipulatorarme, von den vorzugweise einer mit einem Endoskop verbunden wird und die weiteren Manipulatorarme mit Instrumenteneinheiten mit chirurgischen Instrumenten mit Endeffektoren zum Durchführen des chirurgischen Eingriffs. Insbesondere die für die Instrumenteneinheiten mit chirurgischen Instrumenten mit Endeffektoren vorgesehenen Manipulatorarme werden vorzugsweise nacheinander mit Hilfe der Positionierhilfseinheit positioniert und ausgerichtet, um nachfolgend die für den chirurgischen Eingriff erforderlichen Manipulationen vorzunehmen. Die Koordinaten des Zielgebiets können dabei vorzugsweise einen räumlichen Bereich angeben, sodass das für die Einrichtung der Manipulatorarme relevante Zielgebiet eine räumliche Dimension hat. Auch kann für jedes chirurgisches Instrument ein separates Zielgebiet vorgesehen sein, wobei sich die Zielgebiete zumindest teilweise überlappen können.

Die Positionierhilfseinheit umfasst vorzugsweise eine elektronische Schaltung mit einer Sender- und/oder Empfängereinheit, durch die Informationen zu einer Steuereinheit der Vorrichtung zur robotergestützten Chirurgie und/oder von der Steuereinheit der Vorrichtung zur robotergestützten Chirurgie zur Positionierhilfseinheit zu übertragen, um:

- es automatisch als Positionierhilfseinheit zu erkennen,
- die Verwendung der Positionierhilfseinheit in einem sterilen Operationsumfeld bei einer einzigen Operation sicherzustellen, wobei eine einzige Positionierhilfseinheit zur Positionierung mehrerer Manipulatorarme bei einer Operation genutzt werden kann,
- Steuerinformationen zur Positionierhilfseinheit und/oder zur Steuereinheit zu übertragen.

Der Positionierhilfseinheit kann eine eigene Energiequelle, wie z. B. ein Akkumulator oder eine Batterie haben, um die elektronische Schaltung mit Energie zu versorgen, oder sie besitzt elektrische Kontakte für eine elektrische Verbindung mit der Koppeleinheit des Manipulatorarms und/oder enthält Koppelspulen und/oder Antennen, um Energie von der Koppeleinheit des Manipulatorarms zur Positionierhilfseinheit zu übertragen.

Das Licht wird von der Positionierhilfseinheit vorzugsweise mit Hilfe einer Projektionsvorrichtung, wie beispielsweise einer Strahlformoptik, abgestrahlt, die insbesondere ein gewünschtes Lichtmuster, wie z. B. ein Fadenkreuz oder konzentrische Ringe, auf die Oberfläche des Patienten oder auf einen in den Patienten eingeführten Trokar projiziert.

Die Steuereinheit der Vorrichtung zur robotergestützten Chirurgie dient insbesondere

- zur Eingabe und Speicherung der Koordinaten des Zielgebiets im Patientenkoordinatensystem und/oder Koordinatensystem der Vorrichtung,
- zur Berechnung der Orientierung des einzusetzenden Instrumentes aus den Positionen der Segmente des Manipulatorarms
- zur Steuerung der Verwendung der Positionierhilfseinheit, insbesondere zur Erkennung der Positionierhilfseinheit und zur Sicherung gegen Mehrfachverwendung der Positionierhilfseinheit in verschiedenen Operationen,
- zur Ansteuerung der Aktivierung des optischen und/oder akustischen Signals in der Steuereinheit und/oder der Positionierhilfseinheit, wie z. B. der zum Aktivieren der Schaltzustände, Lichtquellen aus, nur Lichtquelle grünes Licht ein, nur Lichtquelle rotes Licht ein, oder nur Lichtquelle weißes Licht ein, nur Lichtquelle grünes Licht ein, nur Lichtquelle rotes Licht ein.

Die elektronische Schaltung der Positionierhilfseinheit kann insbesondere ein RFID-Tag umfassen bzw. durch diesen gebildet werden.

Das Zielgebiet ist insbesondere ein Zieloperationsgebiet. Alternativ oder zusätzlich kann das Zielgebiet durch einen Zielpunkt, wie z.B. durch den Mittelpunkt eines Zieloperationsgebiets oder durch einen von der Lage eines anderen chirurgischen Instruments abhängigen Punkt, festgelegt sein. Wenn das andere chirurgische Instrument ein bereits zumindest zum Teil in den Körper des Patienten eingeführtes Endoskop, wie z.B. ein Stabendoskop, oder ein anderes bildgebendes Systems zum Erfassen von Bildern zumindest eines Ausschnitts eines Zieloperationsgebiets ist, ist es vorteilhaft, wenn das Zielgebiet von der Lage des Endoskops oder des anderen bildgebenden Systems abhängig ist. Beispielsweise kann das Zielgebiet durch einen Punkt auf der optischen Achse der optischen Elemente des Endoskops bzw. der optischen Achse der optischen Elemente des anderen bildgebenden Systems festgelegt sein. Der Zielpunkt ist insbesondere ein Punkt im Schärfentiefebereich, beispielsweise der Brennpunkt oder ein Punkt zwischen dem Brennpunkt und dem proximalen Ende des Endoskops.

Das andere bildgebende System kann insbesondere ein auf nicht sichtbarem Licht basierendes optisches System sein, insbesondere ein Röntgensystem, ein Computertomographiesystem, ein Magnetoresonanz-Tomographiesystem oder ein anderes geeignetes bildgebendes System sein.

Als proximal wird allgemein ein dem Patienten zugewandtes Ende eines beliebigen Elements angesehen. Als distal wird allgemein ein dem Patienten abgewandtes Ende eines beliebigen Elements angesehen.

Weitere Merkmale und Vorteile ergeben sich aus der folgenden Beschreibung, die die Erfindung anhand von Ausführungsbeispielen im Zusammenhang mit den beigefügten Figuren näher erläutert.

Es zeigen:

Figur 1     eine schematische Seitenansicht eines Systems zur robotergestützten Chirurgie mit einem Manipulator, der vier Manipulatorarme hat, mit denen jeweils eine Instrumenteneinheit verbindbar ist;

Figur 2     eine schematische Vorderansicht des Systems nach Figur 1;

Figur 3    eine Anordnung zum Verbinden einer in einem sterilen Bereich einer angeordneten Instrumenteneinheit mit einer nicht sterilen Koppeleinheit eines Manipulatorarms;

Figur 4    eine Positionierhilfseinheit gemäß einer ersten Ausführungsform;

Figur 5    eine Positionierhilfseinheit gemäß einer zweiten Ausführungsform;

Figur 6    die Anordnung nach Figur 3, wobei anstelle der Instrumenteneinheit eine Positionierhilfseinheit nach Figur 4 mit der Koppeleinheit des Manipulatorarms verbunden wird;

Figur 7    einen Abschnitt des Manipulatorarms mit der Koppeleinheit und der mit der Koppeleinheit verbundenen Positionierhilfseinheit mit einer ausgefahrenen Teleskopanordnung des Manipulatorarms gemäß einer ersten Ausführungsform;

Figur 8    die Anordnung nach Figur 7, wobei die Teleskopanordnung eingefahren ist;

Figur 9    die Anordnung nach Figur 7 mit ausgefahrener Teleskopanordnung, wobei anstelle der Positionierhilfseinheit die Instrumenteneinheit mit der Koppeleinheit des Manipulatorarms verbunden ist;

Figur 10   die Anordnung nach Figur 9, wobei die Teleskopanordnung eingefahren ist, sodass das chirurgische Instrument bis in das Zieloperationsgebiet geführt ist oder über diesen hinausgeht;

Figur 11   die schematische Darstellung der Positionierhilfseinheit und des Zieloperationsgebietes in einem Koordinatensystem der Vorrichtung;

Figur 12   eine schematische Darstellung zur Ausrichtung der mit dem Manipulatorarm verbundenen Positionierhilfseinheit zum Zieloperationsgebiet nach einer ersten Vorgehensweise;

Figur 13   eine schematische Darstellung zur Ausrichtung der mit dem Manipulatorarm verbundenen Positionierhilfseinheit zum Zieloperationsgebiet nach einer zweiten Vorgehensweise;

Figur 14   einen Ausschnitt eines Patientenkörpers mit vier markierten Positionen für geplante Körperöffnungen des Patienten, an denen jeweils ein Trokar eingeführt wird;

Figur 15   eine Anordnung mit einem Abschnitt eines Manipulatorarms mit einer Koppeleinheit und der mit der Koppeleinheit verbundenen Positionierhilfseinheit mit eingefahrener Teleskopanordnung des Manipulatorarms gemäß einer zweiten Ausführungsform;

Figur 16   die Anordnung nach Figur 15, wobei eine Trokar-Halterung des Manipulatorarms mit einem in einem Patienten eingeführten Trokar verbunden ist;

Figur 17   die Anordnung nach Figur 16, wobei bei gleicher Ausrichtung des Manipulatorarms und der Koppeleinheit anstelle der Positionierhilfseinheit eine Instrumenteneinheit mit der Koppeleinheit des Manipulatorarms verbunden ist;

Figur 18   eine Anordnung mit einem Abschnitt eines Manipulatorarms mit einer Koppeleinheit und einer mit der Koppeleinheit verbundenen Positionierhilfseinheit mit ausgefahrener Teleskopanordnung des Manipulatorarms gemäß einer dritten Ausführungsform mit einem in den Körper des Patienten eingeführten Trokar zum nachfolgenden Einführen des proximalen Endes eines chirurgischen Instruments und mit einem über einen weiteren Trokar in den Körper des Patienten zumindest teilweise eingeführten Endoskop;

Figur 19   die Anordnung nach Figur 18, wobei die Lage der ausgefahrenen Teleskopanordnung verändert worden ist, so dass die Mittelachse eines von der Positionierhilfseinheit emittierten Strahlenbündels durch ein festgelegtes Zielgebiet im Körper des Patienten verläuft;

Figur 20   die Anordnung nach Figur 19, wobei die Positionierhilfseinheit gegen eine Instrumenteneinheit mit einem chirurgischen Instrument ausgetauscht worden ist ohne die Position der Koppeleinheit des Manipulatorarms zu ändern; und

Figur 21    die Anordnung nach Figur 20 mit eingefahrenen Teleskopanordnung.

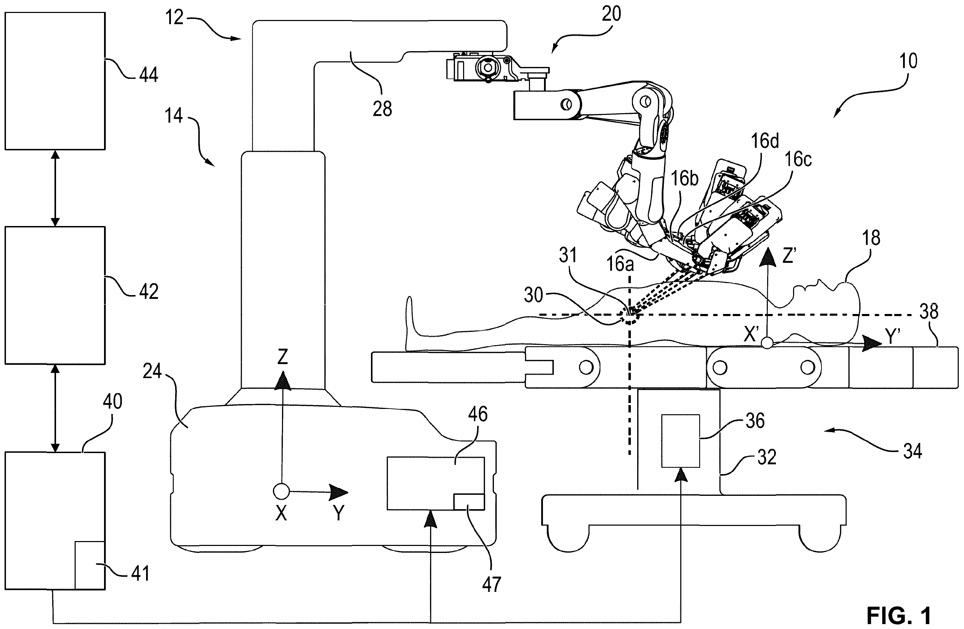

Figur 1 zeigt eine schematische Seitenansicht eines Systems 10 zur robotergestützten Chirurgie mit einem Manipulator 12, der ein Stativ 14 und vier Manipulatorarme 16a bis 16d hat. Der Manipulator 12 wird allgemein auch als Vorrichtung zur robotergestützten Chirurgie bezeichnet. Das System 10 dient zur Durchführung eines chirurgischen Eingriffs an einem auf einem Operationstisch 34 positionierten Patienten 18. Ausgehend von der Anatomie des Patienten 18 und der durchzuführenden Operation sind die Koordinaten $x'_z$, $y'_z$, $z'_z$ eines Zieloperationsgebiets 30 in einem Patientenkoordinatensystem X', Y', Z' ermittelt und diese Koordinaten $x'_z$, $y'_z$, $z'_z$ voreingestellt gespeichert worden. Der Manipulator 12 hat ein Koordinatensystem X, Y, Z der Vorrichtung 12, dessen Koordinatenursprung in einem Stativfuß 24 eines Stativs 14 des Manipulators angeordnet ist. Das Stativ 14 hat einen L-förmigen Stativarm 28, an dessen vom Stativfuß 24 entfernten Ende die Manipulatorarme 16a bis 16d über einen Stativkopf 20 verbunden sind.

Der Operationstisch 34 hat eine Operationstischsäule 32, in der eine Steuereinheit 36 des Operationstischs 34 angeordnet ist und auf der eine mehrere Segmente umfassende Patientenlagerfläche 38 angeordnet ist. Die Steuereinheit 36 dient zur Steuerung der Bewegung von Elementen des Operationstischs 34, insbesondere zur Längenverstellung der Operationstischsäule 32 und somit zum Verstellen der Höhe der Patientenlagerfläche 38 und zum Verstellen einzelner Segmente sowie der Neigung und der Kantung der Patientenlagerfläche 38. Vorzugsweise ist jedoch die Verstellung der Segmente des Operationstischs 34 während eines operativen Eingriffs mit Hilfe des Manipulators 12 gesperrt. Das System 10 umfasst ferner eine Steuereinheit 46 des Manipulators 12 sowie eine zentrale Steuereinheit 40, die über Datenleitungen mit der Steuereinheit 46 des Manipulators 12, der Steuereinheit 36 des Operationstischs 34 sowie einer Bedienkonsole 42 mit einer Anzeigeeinheit 44 verbunden ist. Die Steuereinheit 40 hat eine Ausgabeeinheit 41 und die Steuereinheit 46 hat eine Ausgabeeinheit 47, durch die jeweils optische und/oder akustische Signale ausgegeben werden können.

Die Oberfläche der Patientenlagerfläche 38 bildet eine Frontalebene, auf der der Patient 18 dorsal liegend positioniert ist. Ferner verläuft durch den Koordinatenursprung des Patientenkoordinatensystems X', Y', Z' eine Transversalebene, in der die Koordinatenachsen X' und Z' liegen. Ferner verläuft durch den Koordinatenursprung eine Medianebene, in der die Koordinatenachsen Z' und Y' liegen.

Die Koordinaten $x'_z$, $y'_z$, $z'_z$ des Zieloperationsgebiets 30 in dem Patientenkoordinatensystem X', Y', Z' sind bekannt und können durch die bekannte Lage des Patientenkoordinatensystems X', Y', Z' zum Koordinatensystem X, Y, Z der Vorrichtung 12 einfach bei der Ansteuerung der Manipulatorarme 16a bis 16d sowie der mit den Manipulatorarmen 16a bis 16d verbundenen Instrumenteneinheit zur Durchführung eines chirurgischen Eingriffs mit Hilfe des Manipulators 12 berücksichtigt werden, insbesondere in Koordinaten $x_z$, $y_z$, $z_z$ des Koordinatensystems X, Y, Z der Vorrichtung 12 umgerechnet werden.

Die Position der Koordinaten $y'_z$, $z'_z$ der Mitte des Zieloperationsgebiets 30 sind in Figur 1 in Bezug auf die Koordinatenachsen Y' und Z' mit Hilfe der durch das Zielkoordinatengebiet 30 verlaufenden Strichlinien angedeutet.

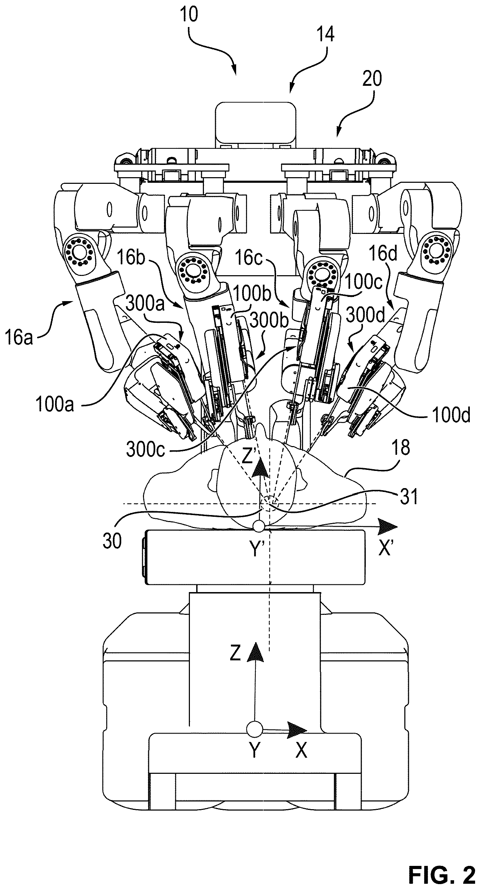

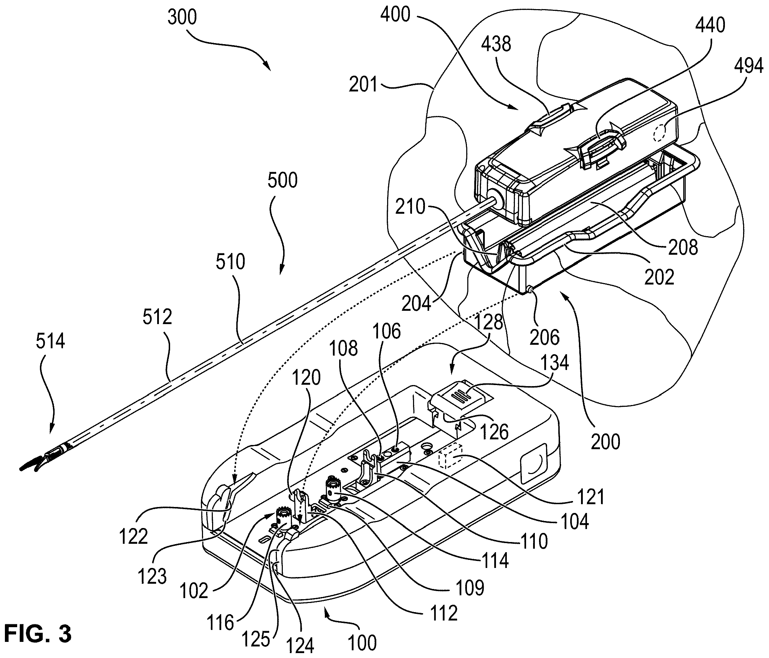

Figur 2 zeigt eine schematische Vorderansicht des Systems 10 nach Figur 1. Am proximalen Ende der Manipulatorarme 16a bis 16d ist jeweils eine Koppeleinheit 100a bis 100d angeordnet, mit den jeweils eine Instrumenteneinheit 300a bis 300d zum Durchführen des chirurgischen Eingriffs verbunden ist. Die Instrumentenschäfte des jeweiligen chirurgischen Instruments der Instrumenteneinheiten 300a bis 300d sind durch Strichlinien angedeutet, die in Figur 2 von den Koppeleinheiten 100a, 100b, 100c, 100d und den mit den Koppeleinheiten 100a, 100b, 100c, 100d verbundenen in Figur 3 dargestellten Sterileinheiten 400a, 400b, 400c, 400d der Instrumenteneinheiten 300a, 300b, 300c, 300d bis ins Zieloperationsgebiet 30 reichen. Die Strichlinien geben dabei die Längsachsen bzw. verlängerten Längsachsen der Instrumentenschäfte an. Mit Hilfe der durch das Zieloperationsgebiet 30 verlaufenden und parallel zu den Koordinatenachsen X' und Z' verlaufenden Strichlinien sind die Koordinaten $y'_z$, $z'_z$ des Mittelpunkts 31 des Zieloperationsgebiets 30 in Bezug auf die Koordinatenachsen X' und Z' angedeutet.

Im Folgenden wird unter Bezugnahme auf den Manipulatorarm 16a die Kopplung der Instrumenteneinheit 300a über eine Sterilschleuse 200a mit der Koppeleinheit 100a des Manipulatorarms 16a beschrieben. Die Ausführungen gelten in gleicher Weise für die weiteren Manipulatorarme 16b bis 16d und für die mit diesen Manipulatorarmen 16b bis 16d über Sterilschleusen 200b bis 200d verbundene Instrumenteneinheiten 300b bis 300d. Zur Vereinfachung werden nachfolgend die Bezugszeichenziffern ohne den zur Unterscheidung zwischen den einzelnen Manipulatorarmen verwendeten Kleinbuchstaben verwendet.

Figur 3 zeigt die Koppeleinheit 100 des Manipulatorarms 16, die Sterilschleuse 200 sowie die Instrumenteneinheit 300 mit der Sterileinheit 400 und einem chirurgischen Instrument 500, das einen Endeffektor 514 hat. Die Koppeleinheit 100, die Sterilschleuse 200 sowie die Instrumenteneinheit 300 sind vor dem Zusammenfügen der Sterilschleuse 200 mit der Koppeleinheit 100 und vor dem nachfolgenden Zusammenfügen der Sterileinheit 400 mit der Sterilschleuse 200 gezeigt. Eine als Sterilfolie 201 ausgeführte flexible sterile

Hülle ist an einem umlaufenden Anschlussrand 202 der Sterilschleuse 200 mit dieser über eine geeignete Verbindung, wie eine Klemm-, Klebe-, Klett- und/oder Schweißverbindung fest verbunden, sodass die Sterilfolie 201 zusammen mit der Sterilschleuse 200 eine geschlossene sterile Abdeckung um die in einen sterilen Operationsbereich ragenden Bereiche des Manipulatorarms 16 bildet.

Zur besseren Darstellung ist in Figur 3 nur ein Ausschnitt der Sterilfolie 201 um die Sterilschleuse 200 herum dargestellt. In den nachfolgenden Figuren ist die Sterilfolie 201 teilweise nicht dargestellt.

Zur Kopplung der Sterileinheit 400 mit der Koppeleinheit 100 ist die Sterilschleuse 200 zwischen der Sterileinheit 400 und der Koppeleinheit 100 angeordnet und ermöglicht im gekoppelten Zustand der Sterileinheit 400 mit der Koppeleinheit 100 eine direkte Kopplung eines ersten Übertragungsmittels 102 der Koppeleinheit 100 und eines zweiten nicht dargestellten Übertragungsmittels der Sterileinheit 400.

Mit Hilfe des ersten Übertragungsmittels 102 wird im vorliegenden Ausführungsbeispiel sowohl mechanische Energie als auch elektrische Energie zwischen der Koppeleinheit 100 und der Sterileinheit 400 übertragen. Hierzu hat das erste Übertragungsmittel 102 der Koppeleinheit 100 beispielsweise mindestens vier mechanische Antriebselemente 110 bis 116 und das zweite Übertragungsmittel 406 der Sterileinheit 400 vier zu den Antriebselementen 110 bis 116 komplementäre angetriebene Elemente 412 bis 418. Weiterhin hat das erste Übertragungsmittel 102 ein elektrisches Übertragungselement 104 mit zwei elektrischen Kontakten 106, 108 und das zweite Übertragungsmittel ein zum elektrischen Übertragungselement 104 des ersten Übertragungsmittels 102 komplementäres elektrisches Übertragungselement.

Das erste Übertragungselement 102 umfasst ferner ein optisches Übertragungsmittel 109 zur Übertragung von Licht und/oder optischen Signalen. Die Antriebselemente des ersten Übertragungsmittels 102 umfassen ein erstes translatorisches Antriebselement 110 und ein zweites translatorisches Antriebselement 112 jeweils zur Übertragung einer translatorischen Bewegung sowie ein erstes rotatorisches Antriebselement 114 und ein zweites rotatorisches Antriebselement 116 zur Übertragung einer Rotationsbewegung. Das erste und das zweite translatorische Antriebselement 110, 112 sind jeweils als Linearhubgabel und das erste und das zweite rotatorische Antriebselement 114, 116 sind als Antriebsritzel mit stirnseitiger Zahnung ausgebildet. Ferner hat die Koppeleinheit 100 einen in einer Vertiefung angeordneten Koppelsensor, der ein durch einen aus der Sterileinheit 400 vorstehenden ersten Detektionsstift gebildetes erstes Detektionselement detektiert, wenn die Sterilschleuse 200 korrekt mit der Koppeleinheit 100 und die Sterileinheit 400 korrekt mit der Sterilschleuse 200 gekoppelt ist.

Bei anderen Ausführungsbeispielen können die ersten und zweiten Übertragungsmittel auch mehr oder weniger Antriebselemente, angetrieben Elemente und elektrische Übertragungselemente umfassen, die mechanische und/oder elektrische Energie durch direkte Kopplung übertragen. Als direkte Kopplung wird dabei eine Kopplung der Übertragungsmittel angesehen, bei der keine weiteren Übertragungselemente zwischen den ersten Übertragungsmitteln und den zweiten Übertragungsmitteln für eine Übertragung von mechanischer und/oder elektrischer Energie und/oder optischen Strahlen vorgesehen sind, wobei insbesondere keine elektrischen, mechanischen oder optischen Übertragungselemente in einer zwischen der Koppeleinheit 100 und der Sterileinheit 400 angeordneten Sterilbarriere, wie der Sterilschleuse 200, vorgesehen sind. Die Koppeleinheit 100 hat ferner eine RFID-Lese-und-Schreibeinheit 121, mit deren Hilfe ein RFID-Transponder 494 der Sterileinheit 400 lesbar und/oder schreibbar ist.

Zum Verbinden der Koppeleinheit 100 mit der Sterilschleuse 200 hat die Koppeleinheit 100 einander gegenüberliegende Führungsnuten 122, 124, in die Führungsstifte 204, 206 der Sterilschleuse 200 eingeführt werden, bis sie das vordere Ende 123, 125 der jeweiligen Führungsnut 122, 124 erreicht haben. Die Führungsstifte 204, 206 ragen an einem ersten Ende der Sterilschleuse 200 auf gegenüberliegenden Seiten nach außen. Anschließend wird das gegenüberliegende zweite Ende der Sterilschleuse 200 nach unten gedrückt, so dass die Sterilschleuse 200 um eine durch die Führungsstifte 204, 206 verlaufende Drehachse gedreht wird, bis eine Rastnase 126 eines Rastelements 128 in einen komplementären Rastbereich der Sterilschleuse 200 eingreift.

Die Entriegelungstaste 134 ist um eine Drehachse schwenkbar angeordnet und wird durch eine Feder in ihrer in Figur 3 gezeigten Rastposition gehalten. Zum Lösen der Rastverbindung wird mit einem Finger auf eine Entriegelungstaste 134 des Rastelements 128 gedrückt, so dass eine Feder gespannt und das Rastelement 128 zusammen mit der Rastnase 126 verschwenkt wird, so dass die Rastnase 126 außer Eingriff mit dem komplementären Rastelement der Sterilschleuse 200 gebracht wird. Dadurch kann das zuvor mit der Rastnase 126 in Eingriff stehende zweite Ende der Sterilschleuse 200 aus der Koppeleinheit 100 wieder herausgeschwenkt werden. Nachdem dieses zweite Ende der Sterilschleuse 200 aus der Koppeleinheit 100 herausgeschwenkt worden ist, kann die Sterilschleuse 200 wieder vollständig von der Koppeleinheit 100 getrennt werden, indem die Sterilschleuse 200 mit den mit den Führungsnuten 122, 124 in Eingriff stehenden Führungsstiften 204, 206 entlang der Führungsnuten 122, 124 aus diesem heraus gezogen wird, bis die Führungselemente 204, 206 nicht mehr in Eingriff mit den Führungsnuten 122, 124 stehen. Zwischen den Führungsnuten 122, 124 und dem Rastelement 128 ist ein durch eine entsprechende Vertiefung im Gehäuse der Koppeleinheit 100 gebildeter Aufnahmebereich vorhanden, der im vorliegenden Ausführungsbeispiel die Sterilschleuse 200 auf drei Seiten und bodenseitig zumindest teilweise umgibt.

Die Sterilschleuse 200 hat Schleusenklappen 208, 210, die über Scharniere schwenkbar sind. Mit Hilfe dieser Scharniere sind die Schleusenklappen 208, 210 von dem in Figur 3 gezeigten geschlossenen Zustand in einen geöffneten Zustand schwenkbar. Im geöffneten Zustand der Schleusenklappen 208, 210 kann eine direkte Kopplung des ersten Übertragungsmittels 102 der Koppeleinheit 100 mit dem zweiten Übertragungsmittel der Sterileinheit 400 erfolgen.

An den Außenseiten der Seitenwände und der Stirnwände der Sterilschleuse 200 ist ein umlaufender Rand 202 ausgebildet, mit dem die Sterilfolie 201 der sterilen Abdeckung auf geeignete Art und Weise verbunden ist.

Die Sterileinheit 400 hat ferner gegenüberliegend angeordnete Rast- und Betätigungselemente 438, 440 durch die automatisch eine wieder lösbare Rastverbindung beim Verbinden der Sterileinheit 400 mit der Sterilschleuse 200 erfolgt.

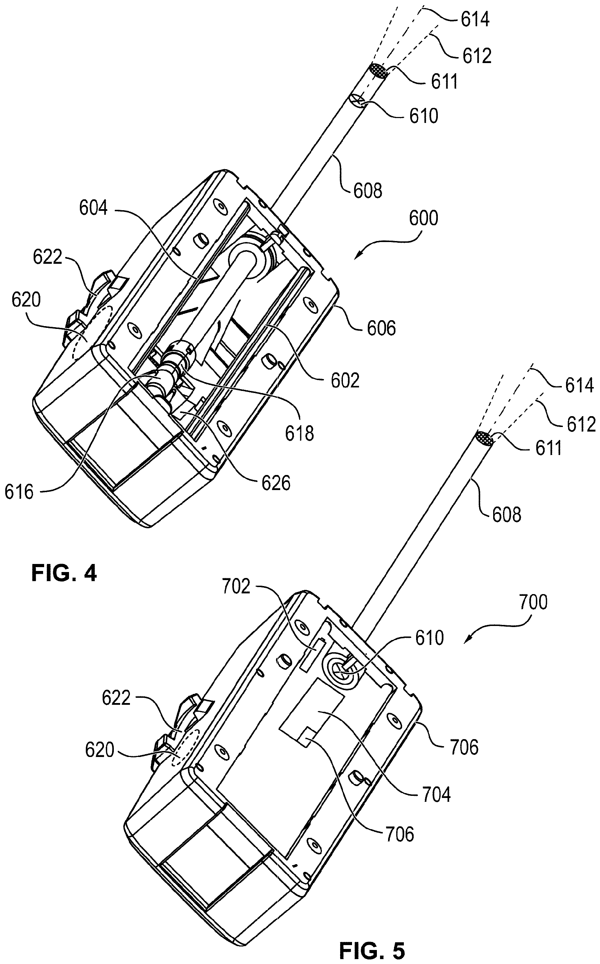

Figur 4 zeigt eine Positionierhilfseinheit 600 gemäß einer ersten Ausführungsform, die anstelle der Instrumenteneinheit 300 mit der Sterilschleuse 200 zum Positionieren des Manipulatorarms 16 vor einem geplanten chirurgischen Eingriff an einem Patienten über die Sterilschleuse 200 mit der Koppeleinheit 100 des Manipulatorarms 16 verbunden wird.

Die Positionierhilfseinheit 600 hat Sterilklappen 602, 604, die beim Verbinden der Positionierhilfseinheit 600 mit der Sterilschleuse 200 automatisch entriegelt und geöffnet werden, bis sie in dem in Figur 4 gezeigten geöffneten Zustand sind. Beim Trennen der Positionierhilfseinheit 600 von der Sterilschleuse 200 werden die Sterilklappen 602, 604 automatisch geschlossen, vorzugsweise mit Hilfe der Federkraft mindestens einer Feder, und anschließend verriegelt, sodass die Elemente im Inneren der Positionierhilfseinheit 600 mit Hilfe der Sterilklappen 602, 604 steril abgedeckt sind.

Das Gehäuse 606 der Positionierhilfseinheit 600 ist in diesem Ausführungsbeispiel identisch mit dem Gehäuse der Sterileinheit 400 der Instrumenteneinheit 300. Bei anderen Ausführungsformen kann sich das Gehäuse 606 auch in Größe und Form unterscheiden. Die Positionierhilfseinheit 600 hat eine in einem aus dem Gehäuse 606 der Positionierhilfseinheit 600 ragenden Schaft 608 angeordnete Lichtquelle 610. Das Licht der Lichtquelle 610 wird als Strahlenbündel 612 mit sichtbarem Licht durch ein strahlformendes optisches Element 611 von der Spitze des Schafts 608 als Strahlenbündel 612 emittiert, dessen Mittelachse 614 mit der Längsachse des Schafts 608 übereinstimmt.

Die Positionierhilfseinheit 600 hat zwei elektrische Kontakte 616, 618, die bei geöffneten Sterilklappen 602, 604 eine elektrisch leitend mit den Kontakten 106, 108 der Koppeleinheit 100 in direkter Verbindung stehen. Über diese Kontakte 616, 618 wird die Lichtquelle 610 mit elektrischer Energie versorgt und angesteuert. Bei anderen Ausführungsformen können auch drei oder mehr elektrische Kontakte 106, 108 der Koppeleinheit 100 und drei oder mehr elektrische Kontakte 616, 618 der Positionierhilfseinheit 600 vorgesehen sein, um mehrere Lichtquellen 610, die Licht unterschiedlicher Wellenlängen emittieren, anzusteuern. Alternativ kann die Wellenlänge des abgegebenen Lichts einer einzigen Lichtquelle 610 durch eine entsprechende Ansteuerschaltung in der Positionierhilfseinheit 600 bewirkt werden, die beispielsweise durch unterschiedliche Potentialdifferenzen zwischen den elektrischen Kontakten 616, 618 unterschiedliche Lichtquellen 610 oder eine Lichtquelle 610 so ansteuert, dass diese Licht unterschiedlicher Wellenlägen abgibt. So ist es beispielsweise möglich, dass bei einer Potentialdifferenz von 5 V zwischen den elektrischen Kontakten 616, 618 rotes Licht durch die Lichtquelle 610 und bei einer Potentialdifferenz von 12 V zwischen den elektrischen Kontakten 616, 618 grünes Licht durch die Lichtquelle 610 abgestrahlt wird. Die Lichtquelle 610 kann insbesondere einen oder mehrere Laser, eine oder mehrere LEDs, insbesondere eine Mehrfarben-LED, oder eine oder mehrere Glühlampen umfassen. Im einfachsten Fall strahlt die Lichtquelle 610 kontinuierlich Licht mit einer konstanten Wellenlänge bzw. mit einem konstanten Wellenlängenspektrum ab. Weiterhin hat die Positionierhilfseinheit 600 eine Ausgabeeinheit 626, durch die alternativ oder zusätzlich akustische Signale ausgegeben werden können.

Ferner hat die Positionierhilfseinheit 600 einen RFID-Transponder 620, der mit Hilfe der RFID-Lese- und Schreibeinheit 121 der Koppeleinheit lesbar und/oder beschreibbar ist. Insbesondere dient der RFID-Transponder 620 in Verbindung mit der RFID-Lese- und Schreibeinheit 121 und der Steuereinheit 46 zur Überwachung und Unterbindung der Mehrfachverwendung der Positionierhilfseinheit 600 bei mehreren Operationen. In gleicher Weise wie die Sterileinheit 400 hat die Positionierhilfseinheit 600 zwei gegenüberliegend angeordnete Rast- und Betätigungselemente 622, 624, durch die eine lösbare Rastverbindung zwischen der Positionierhilfseinheit 600 und der Sterilschleuse 200 herstellbar ist. Die Überwachung der korrekten Verbindung der Positionierhilfseinheit 600 mit der Sterilschleuse 200 und der Koppeleinheit 100 mit Hilfe des Koppelsensors 120 erfolgt in gleicher Weise wie in Verbindung mit der Sterileinheit 400 bereits beschrieben.

Figur 5 zeigt eine Positionierhilfseinheit 700 gemäß einer zweiten Ausführungsform. Die Positionierhilfseinheit 700 ist in gleicher Weise wie die Positionierhilfseinheit 600 anstelle der Instrumenteneinheit 300 über die Sterilschleuse 200 mit der Koppeleinheit 120 verbindbar. Im Unterschied zu der Positionierhilfseinheit 600 nach Figur 4 hat die Positionierhilfseinheit 700 keine Sterilklappen und damit auch keinen Mechanismus zu deren Betätigung. Elemente der Positionierhilfseinheit 700, die in Aufbau und/oder Funktion mit den Elementen der Positionierhilfseinheit 600 übereinstimmen, haben dieselben Bezugszeichen. Die Positionierhilfseinheit 700 hat zusätzlich eine Energiequelle 702 in Form einer Batterie und eine elektronische Schaltung 704, die insbesondere einen Controller zur Ansteuerung der Lichtquelle 610 umfassen kann. Die elektronische Schaltung 704 umfasst eine Ausgabeeinheit 706, durch die optische und/oder akustische Signale ausgegeben werden können.

Die Energiequelle 702 versorgt sowohl die elektrische Schaltung 704 als auch die Lichtquelle 610 mit elektrischer Energie. Ferner ist eine drahtlose Datenverbindung zwischen der elektronischen Schaltung 704 und der Steuereinheit 46 des Manipulators 12 zur Übertragung von Steuerinformationen vorgesehen. Bei alternativen Ausführungsformen wird die Steuerinformation über den RFID-Transponder 620 zur elektronischen Schaltung 704 übertragen. Somit erfolgt kein direkter Kontakt von Elementen der Positionierhilfseinheit 700 mit nicht sterilen Elementen, insbesondere Übertragungselementen 102 der Koppeleinheit 100, sodass bei einer Verbindung der Positionierhilfseinheit 700 kein Element der Positionierhilfseinheit 700 durch nicht sterile Elemente der Koppeleinheit 100 kontaminiert werden, sodass die Sterilklappen 602, 604 bei dieser Ausführungsform zur Sicherstellung einer sterilen Handhabung der Positionierhilfseinheit 700 nicht erforderlich sind. Bei der Ausführungsform der Positionierhilfseinheit 600 nach Figur 4 ist eine sterile Abdeckung der elektrischen Kontakte 616, 618 nach dem Trennen der Positionierhilfseinheit 600 von der Sterilschleuse 200 erforderlich, da die elektrischen Kontakte 616, 618 mit den nicht sterilen elektrischen Kontakten 106, 108 der Koppeleinheit 100 in Kontakt waren und somit kontaminiert worden sind.

Der weitere Aufbau und Funktion des Gehäuses 606 der Positionierhilfseinheit 600 und des Gehäuses 706 der Positionierhilfseinheit 700 stimmen mit der Sterileinheit 400 überein.

Die Mittelachse 614 des Strahlenbündels 612 der Positionierhilfseinheiten 600, 700 stimmen mit der Mittelachse 510 des Instrumentenschafts 512 in Lage und Ausrichtung mit der Mittelachse 510 des Instrumentenschafts 512 überein, wenn anstelle der Instrumenteneinheit 300 die Positionierhilfseinheit 600 bzw. Positionierhilfseinheit 700 über die Sterilschleuse 200 mit der Koppeleinheit 100 verbunden ist.

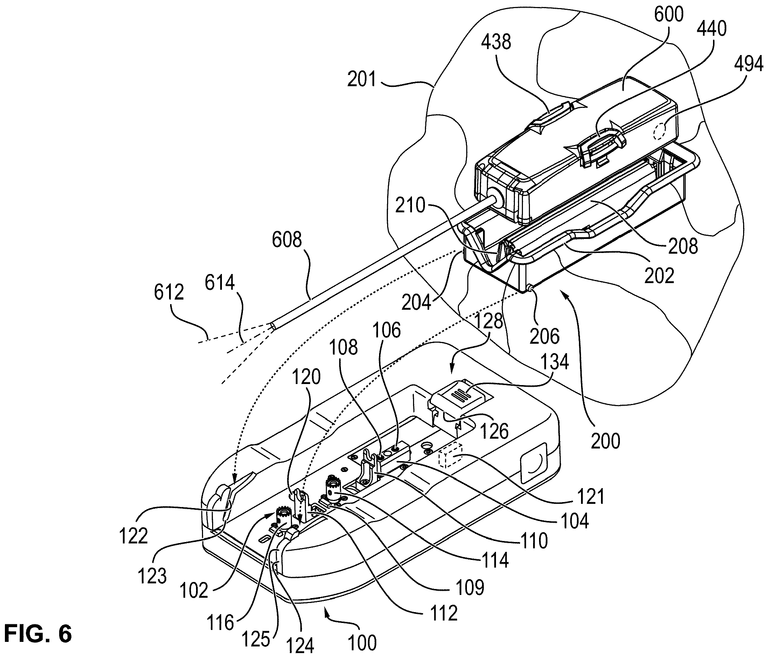

Figur 6 zeigt die Koppeleinheit 100 des Manipulatorarms 16, die Sterilschleuse 200 sowie die Positionierhilfseinheit 600. Die Koppeleinheit 100, die Sterilschleuse 200 sowie die Positionierhilfseinheit 600 sind vor dem Zusammenfügen der Sterilschleuse 200 mit der Koppeleinheit 100 und vor dem nachfolgenden Zusammenfügen der Positionierhilfseinheit 600 mit der Sterilschleuse 200 gezeigt. Wie bereits in Verbindung mit den Figuren 4 und 5 erläutert, stimmt die Mittelachse 614 des von der Positionierhilfseinheit 600 emittierten Strahlenbündels 612 mit der Längsachse 510 des Instrumentenschafts 512 in Lage und Ausrichtung überein, sodass bei einer Projektion des Strahlenbündels 612 auf eine Oberfläche der Schnittpunkt der Längsachse 510 des Instrumentenschafts 512 mit der Projektionsfläche grafisch angezeigt werden kann. Die Lage und Position der Längsachse 510 des Instrumentenschafts 512 des chirurgischen Instruments 500 stimmt nach einem Tausch der mit der Koppeleinheit 100 über die Sterilschleuse 200 verbundenen Positionierhilfseinheit 600 gegen die Instrumenteneinheit 300 mit der Mittelachse 614 des Strahlenbündels 612 überein, wenn bei diesem Tausch die Lage und Ausrichtung der Koppeleinheit 100 des Manipulatorarms 16 unverändert bleibt.

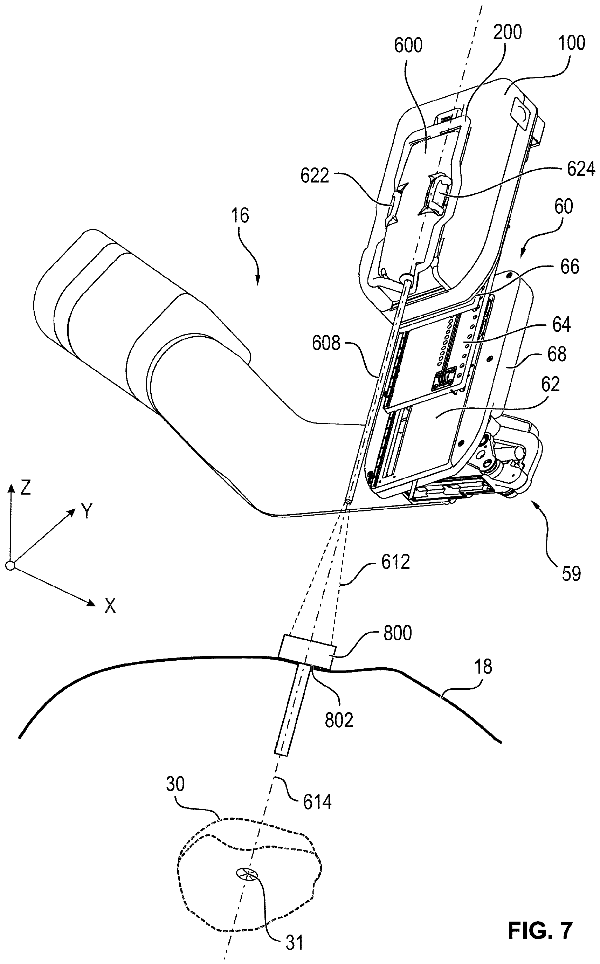

Figur 7 zeigt einen Abschnitt des Manipulatorarms 16 an dessen proximalem Ende die Koppeleinheit 100 über eine Teleskopanordnung 60 verbunden ist. Die Teleskopanordnung 60 hat zueinander verschiebbare Abschnitte 62, 64, 66 und ist in Figur 7 in einem ausgefahrenen Zustand dargestellt. Die Abschnitte 62, 64, 66 der Teleskopanordnung 60 können mit Hilfe einer Antriebseinheit 68 eingefahren und ausgefahren werden, sodass die Spitze des Schafts 608 der Positionierhilfseinheit 600 entlang der Längsachse des Schafts 608 bzw. der Mittelachse 614 des von der Positionierhilfseinheit 600 emittierten Strahlenbündels 612 bewegt werden kann. Der Manipulatorarm 16 hat mehrere zueinander bewegbare Segmente, deren Position relativ zueinander geändert werden kann. Die Teleskopanordnung 60 ist ferner über ein Koppelgetriebe 59 mit den weiteren Segmenten des Manipulatorarms 16 schwenkbar verbunden, sodass nach einem Austausch der Positionierhilfseinheit 600 gegen die Instrumenteneinheit 300 die Lage und Ausrichtung der Längsachse 510 des Instrumentenschafts 512 des chirurgischen Instruments 500 in seiner Position, d. h. sowohl in seiner Ausrichtung als auch in seiner Lage, durch eine Bedieneingabe an der Bedienkonsole 42 geändert werden kann. Zum Einrichten jedes Manipulatorarms 16 vor einer Operation ist die Koppeleinheit 100 derart auszurichten, dass die Längsachse 510 des Instrumentenschafts 512 eines mit der Koppeleinheit 100 verbundenen chirurgischen Instruments 500 durch eine geplante oder vorhandene Körperöffnung 802 des zu operierenden Patienten 18 hindurch und durch ein festgelegtes Zieloperationsgebiet 30 verläuft. In Figur 7 ist an der Körpereintrittsstelle 802 ein Trokar 800 in den Körper des Patienten 18 eingeführt, durch den dann zum Durchführen des chirurgischen Eingriffs der vordere Teil des Schafts 512 des chirurgischen Instruments 500 zusammen mit dem Endeffektor 514 bis zum Zieloperationsgebiet 30 geführt wird. Das von der Positionierhilfseinheit 600 emittierte Strahlenbündel 612 wird dabei durch den Manipulator 12 automatisch oder durch eine Bedienperson so ausgerichtet, dass dessen Mittelachse 614 durch die Öffnung des Trokars 800 verläuft. Dabei kann mit Hilfe des strahlformenden optischen Elements 611 ein Hilfsmuster, wie beispielsweise ein Fadenkreuz oder mehrere konzentrische Ringe, um die Mittelachse 614 des Strahlenbündels 612 herum gebildet werden, die eine mittige Ausrichtung des Strahlenbündels 612 auf die gewünschte oder vorhandene Körpereintrittsstelle 802 vereinfacht. Ferner ermittelt die Steuereinheit 46 des Manipulators und/oder die zentrale Steuereinheit 40 den Betrag des Abstandsvektors zwischen der Mittelachse 614 des emittierten Strahlenbündels 612 zum Zieloperationsgebiet 30, insbesondere den Betrag des orthogonalen Abstandsvektors von der Mittelachse 614 zum Zieloperationsgebiet 30. Dabei ist es möglich, sowohl den Betrag des Abstandsvektors zum Rand des Zieloperationsgebiets 30 und alternativ oder zusätzlich den Betrag des Abstandsvektors zum Mittelpunkt 31 des Zieloperationszielgebiets 30 zu bestimmen.

Im vorliegenden Ausführungsbeispiel verläuft die Mittelachse 614 durch den Mittelpunkt 31 des Zieloperationsgebiets 30, sodass der Betrag des Abstandsvektors im vorliegenden Ausführungsbeispiel zwischen Mittelachse 614 und dem zum Zieloperationsgebiet 30 Null beträgt, da die Mittelachse 614 durch das Zieloperationsgebiet 30 verläuft. Auch zum Mittelpunkt 31 des Zieloperationsgebiets 30 beträgt der Abstand Null, da die Mittelachse 614 durch den Mittelpunkt 31 des Zieloperationsgebiets 30 verläuft. Wenn der Betrag des orthogonalen Abstandsvektors einen ersten Wert unterschreitet, kann einer Bedienperson ein erstes optisches und/oder akustisches Signal und wenn der Betrag des Abstandsvektors einen zweiten Wert erreicht oder unterscheidet ein zweites optisches und/oder akustisches Signal ausgegeben werden. Der zweite Wert kann insbesondere Null sein, sodass das zweite optische und/oder akustische Signal dann ausgegeben wird, wenn die Mittelachse 614 durch das Zieloperationsgebiet 30 oder dessen Mittelpunkt 31 verläuft. Auch kann sich das ausgegebene optische und/oder akustische Signal abhängig vom ermittelten Betrag des Abstandsvektors kontinuierlich mit Änderungen des Abstands oder in mehreren Schritten ändern, sodass der Bedienperson akustisch oder optisch informiert wird, ob sich die Mittelachse 614 vom Zieloperationsgebiet 30 entfernt oder sich diesem annähert.

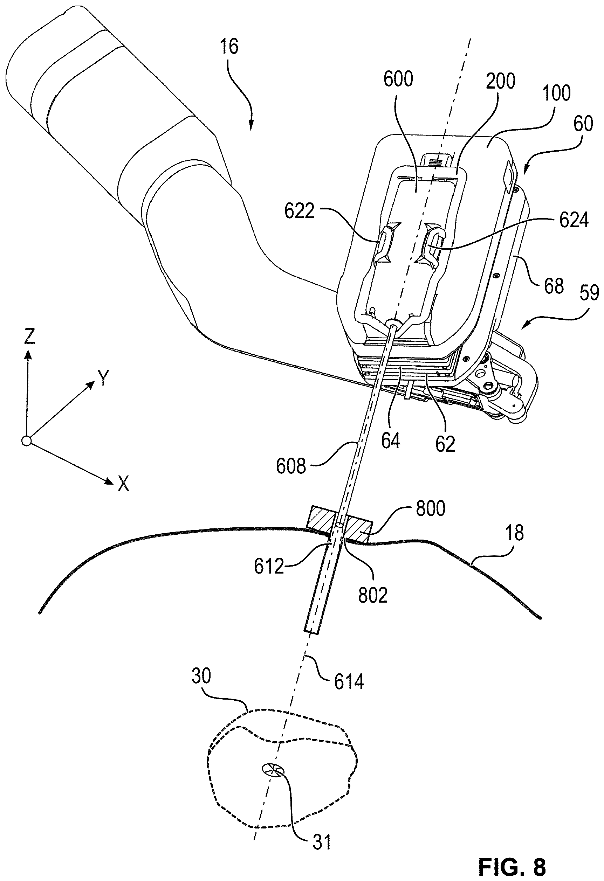

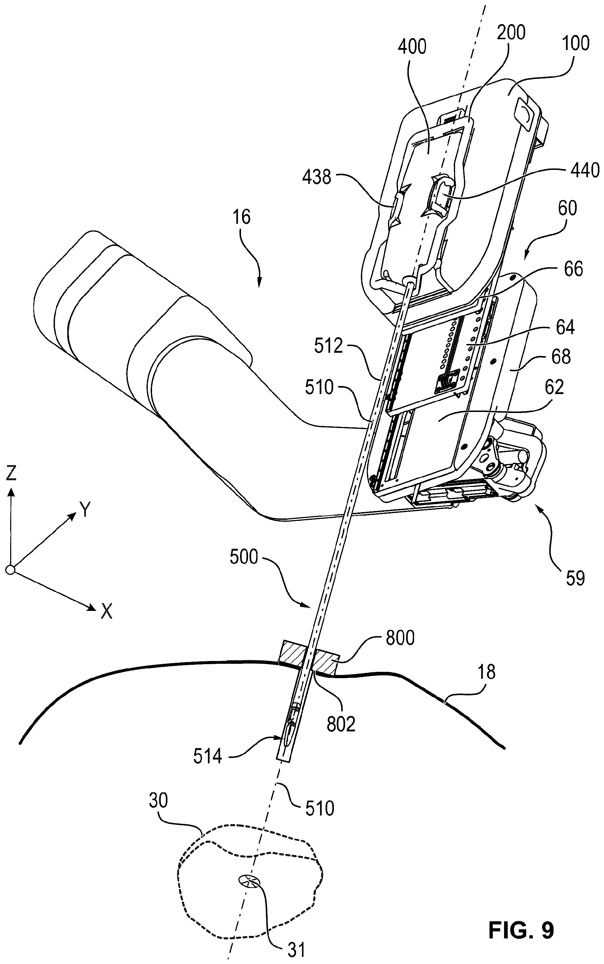

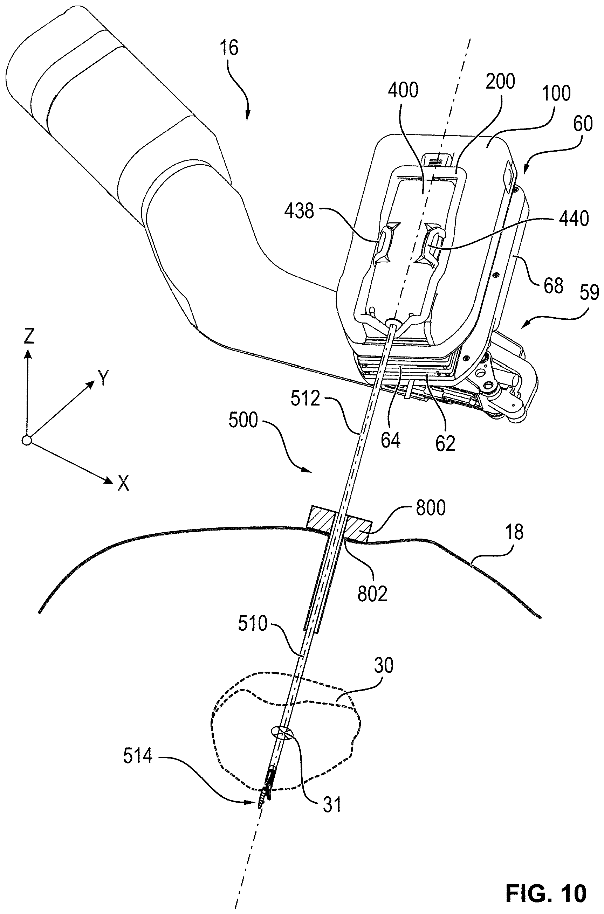

Figur 8 zeigt die Anordnung nach Figur 7, wobei die Abschnitte 62 bis 66 der Teleskopanordnung 60 im Unterschied zu Figur 7 in einem eingefahrenen Zustand dargestellt sind. Der Manipulatorarm 16 wird beim Einrichten vorzugsweise so positioniert, dass die Spitze des Schafts 608 der Positionierhilfseinheit 600 in den in den Körper des Patienten 18 eingeführten Trokars 800 ragt, wenn die Teleskopanordnung 60 eingefahren ist. Dabei wird der Manipulatorarm 16 so positioniert, dass die Spitze des Schafts 608 vorzugsweise mit einer Länge im Bereich von 0,5 cm bis 5 cm, vorzugsweise in einem Bereich zwischen 0,7 cm und 2 cm, insbesondere 1 cm, in den Trokar 800 ragt. Wird dann die Teleskopanordnung 60 ausgefahren, wie dies in Figur 7 gezeigt ist, kann die Positionierhilfseinheit 600 durch Betätigen der Rast- und Betätigungselemente 622, 624 von der Sterilschleuse 200 getrennt und aus dieser entnommen werden und anstelle der Positionierhilfseinheit 600 die Sterileinheit 400 der Instrumenteneinheit 300 in die Sterilschleuse 200 eingesetzt und mit dieser verbunden werden. Die Längen des Schafts 608 der Positionierhilfseinheit 600 und des Instrumentenschafts 512 sind dabei derart aufeinander abgestimmt, dass der Endeffektor 514 des chirurgischen Instruments 500 dann mit einer vorzugsweise geringen Länge innerhalb des Trokars 800 angeordnet ist, wie dies in Figur 9 gezeigt ist. Die Länge hat vorzugsweise einen Wert im Bereich von 2 cm bis 6 cm und beträgt insbesondere 4 cm. Anschliekann die Teleskopanordnung 60 eingefahren werden, sodass der Endeffektor 514 des chirurgischen Instruments 500 durch den Trokar 800 bis in das Zieloperationsgebiet 30 geführt wird. Wie in Figur 10 gezeigt ist, kann der Endeffektor 514 auch durch das Zieloperationsgebiet 30 in Richtung der Längsachse 510 des Instrumentenschafts 512 hindurch und über das Zieloperationsgebiet 30 hinaus bewegt werden.

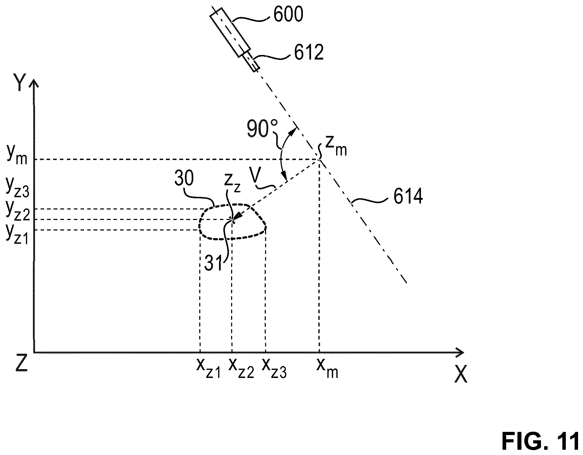

Figur 11 zeigt die schematische Darstellung der Positionierhilfseinheit 600 und des Zieloperationsgebiets 30 in dem Koordinatensystem X, Y, Z der Vorrichtung bzw. des Manipulators 12. Die räumliche Ausdehnung des Zieloperationsgebiets 30 wurde mit Hilfe eines geeigneten bildgebenden Verfahrens für den konkreten Patienten 18 ermittelt und kann dabei als einfache geometrische Körper, wie durch eine Kugel, oder durch die konkrete räumliche Ausdehnung eines für einen speziellen chirurgischen Eingriff an dem Patienten 18 ermitteltes und/oder festgelegtes Zieloperationsgebiet 30 durch eine Vielzahl von Koordinaten festgelegt sein. Das Zieloperationsgebiet 30 kann für einen Patienten 18 insbesondere mit Hilfe eines bildgebenden Verfahrens ermittelt bzw. bei Auswertung der ermittelten Bilder automatisch oder von einer Bedienperson festgelegt werden. Als bildgebendes Verfahren kann ein Röntgenverfahren, ein Computertomographieverfahren, ein Magnetresonanzverfahren oder ein anderes geeignetes Verfahren eingesetzt werden. Die Außenabmessungen des Zieloperationsgebiets 30 sind in den in Figur 11 gezeigten zweidimensionalen Koordinatensystem 26 durch die Koordinaten $x_{zi}$ und $x_{Z3}$ in X-Richtung sowie durch die Koordinaten $y_{Z1}$ und $y_{Z3}$ auf der X-Achse festgelegt. Die Mitte 31 des ermittelten Zieloperationsgebiets 30 ist durch die Koordinate $x_{Z2}$ auf der X-Achse und $y_{Z2}$ auf der Y-Achse festgelegt. In gleicher Weise ist die räumliche Ausdehnung des Zieloperationsgebiets 30 auf der orthogonal zur Bildebene verlaufenden Z-Achse bekannt bzw. festgelegt. Die Koordinaten des Schnittpunkts des Vektors V mit der Mittelachse 614 des Strahlenbündels 612 sind in Fig.11 mit $x_m$, $y_m$, $z_m$ bezeichnet.

Die Mittelachse 614 des von der Positionierhilfseinheit 600 emittierten Strahlenbündels 612 der mit der Koppeleinheit 100 über die Sterilschleuse 200 verbundenen Positionierhilfseinheit 600 stimmt in ihrer Lage und Ausrichtung mit der Längsachse 510 des Instrumentenschafts 512 der anstelle der Positionierhilfseinheit 600 mit der Koppeleinheit 100 über die Sterilschleuse 200 verbundenen Instrumenteneinheit überein. Wie bereits erläutert, ist der Manipulatorarm 16 zusammen mit der Koppeleinheit 100 vor einem chirurgischen Eingriff am Patienten 18 so zu positionieren, dass der von der Positionierhilfseinheit 600 emittierte Strahlenbündel 612 auf eine gewünschte Körperöffnung 802 trifft und die Mittelachse 614 des emittierten Strahlenbündels 612 durch das Zieloperationsgebiet 30 verläuft. Um insbesondere eine Bedienperson bei der korrekten Ausrichtung des Manipulatorarms 16 und der Koppeleinheit 100 zu unterstützen, ermittelt die Steuereinheit 46 den Betrag des zur Mittelachse 614 kürzesten dreidimensionalen orthogonalen Abstandsvektors V zwischen der Mittelachse 614 und dem Mittelpunkt 31 des Zieloperationsgebiets 30. Erreicht oder unterschreitet der Betrag des Abstandsvektors V einen ersten Wert, wird ein erstes optisches und/oder akustisches Signal ausgegeben, erreicht oder unterschreitet er einen zweiten Wert, so wird ein zweites optisches und/oder akustisches Signal ausgegeben, sodass der Bedienperson eine optische und/oder akustische Information über die korrekte Ausrichtung der Koppeleinheit 100 gegeben wird. Hierdurch ist eine einfache und komfortable Möglichkeit geschaffen, eine Bedienperson bei der Einrichtung des Manipulators 12 und bei der Positionierung der Manipulatorarme 16a bis 16d vor dem eigentlichen chirurgischen Eingriff zu unterstützen.

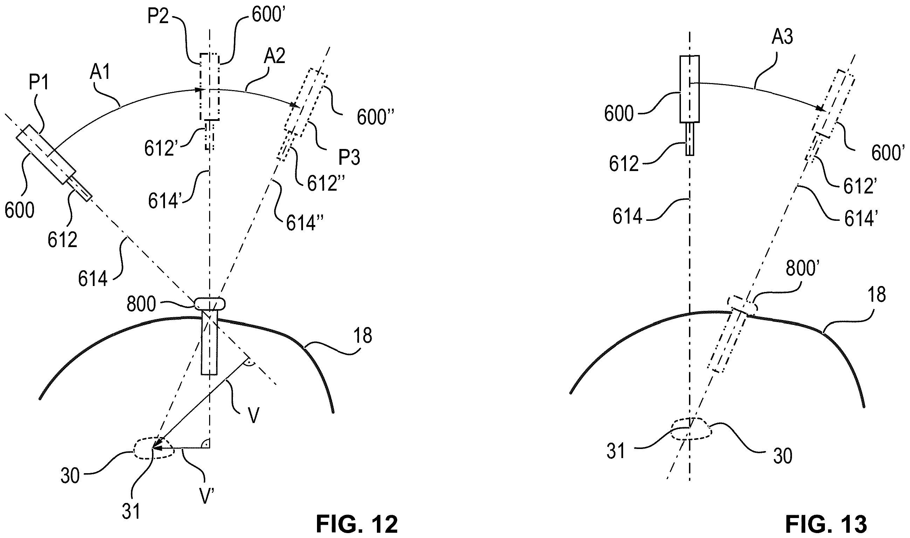

Figur 12 zeigt eine schematische Darstellung zur Ausrichtung der mit der Koppeleinheit 100 verbundenen Positionierhilfseinheit 600 zum Zieloperationsgebiet 30 nach einer ersten Vorgehensweise. Bei dieser Vorgehensweise wird der Manipulatorarm 16 zusammen mit der Koppeleinheit 100 in einem ersten Schritt derart ausgerichtet, dass die Mittelachse 614 des von der Positionierhilfseinheit 600 emittierten Strahlenbündels 612 auf die Instrumenteneinführöffnung des Trokars 800 trifft. Bei dieser Ausrichtung ist der Betrag des Abstandsvektors V größer als ein voreingestellter Wert, sodass die Steuereinheit 46 eine Steuerinformation erzeugt, die angibt, dass die Mittelachse 614 nicht durch das Zieloperationsgebiet 30 verläuft. Der Betrag des Abstandsvektors V ist jedoch so groß, dass er einen ersten voreingestellten Wert überschreitet, sodass die Positionierhilfseinheit 600 lediglich weißes Licht abstrahlt und/oder kein akustisches Signal ausgibt. Wird die Koppeleinheit 100 zusammen mit der Positionierhilfseinheit 600 von der Position P1 in Richtung des Pfeils A1 zur Position P2 verschwenkt, ist der Betrag des Abstandsvektors V' zwischen dem Mittelpunkt 31 des Zieloperationsgebiets 30 und der Mittelachse 614' des von der Positionierhilfseinheit 600' emittierten Strahlenbündels 612' geringer als ein erster voreingestellter Wert, sodass die Positionierhilfseinheit 600 ein erstes akustisches Signal ausgibt und/oder Licht eines anderen Spektrums und/oder eines Teilspektrums des zuvor ausgestrahlten Lichts emittiert, sodass eine Bedienperson an der Farbänderung die Annäherung der Mittelachse 614' an das Zieloperationsgebiet 30 einfach erfassen kann. Dann wird die Koppeleinheit 100 zusammen mit der Positionierhilfseinheit 600' von der Position P2 weiter in Richtung des Pfeils A2 verschwenkt, bis die Positionierhilfseinheit 602' die Position P3 erreicht wird der Betrag des Abstandsvektors V' weiter verringert wird, bis er insbesondere den Wert Null erreicht hat, sodass ein zweiter voreingestellter Wert des Betrags des Abstandsvektors V' erreicht oder unterschritten wird. Ist das der Fall, gibt die Positionierhilfseinheit 600" ein zweites optisches und/oder akustisches Signal ausgibt, durch das die Bedienperson über die korrekte Ausrichtung der Mittelachse 614" in Bezug auf das Zieloperationsgebiet 30 informiert wird. Das zweite optische Signal kann gegenüber dem ersten optischen Signal unterschiedliches Wellenlängenspektrum oder eine unterschiedliche Wellenlänge aufweisen, sodass die Bedienperson durch die Farbänderung über die korrekte Ausrichtung des Manipulatorarms 16 informiert wird.

Alternativ oder zusätzlich kann das zweite optische Signal eine gegenüber dem ersten optischen Signal verschiedene Blinkfrequenz haben. Anschließend kann die Bedienperson die Positionierhilfseinheit 600 von der Sterilschleuse 200 und somit von der Koppeleinheit 100 trennen und anstelle der Positionierhilfseinheit 600 die für diesen Manipulatorarm 16 vorgesehene Instrumenteneinheit 300 mit der Koppeleinheit 100 über die Sterilschleuse 200 verbinden.

Beim Vorsehen von zwei Grenzwerten, mit denen der Betrag des Abstandsvektors V jeweils verglichen wird, sind somit drei Zustände erfassbar, so dass einer Bedienperson bereits bei der Annäherung an das Zieloperationsgebiet 30 ein entsprechendes optisches und/oder akustisches Signal ausgegeben werden kann und einer korrekten Ausrichtung der Positionierhilfseinheit 600 auf das Zieloperationsgebiet 30 ein weiteres optisches und/oder akustisches Signal ausgegeben werden kann. Beim Vorsehen von nur einem Grenzwert können zwei Zustände einfach unterschieden werden, insbesondere das ein Abstand zwischen dem Zieloperationsgebiet 30 und der Mittelachse 614 existiert, d.h. dass die Mittelachse 614 nicht durch das Zieloperationsgebiet 30 verläuft, und dem Zustand, dass die Mittelachse 614 durch das Zieloperationsgebiet 30 verläuft.

Figur 13 zeigt eine schematische Darstellung zur Ausrichtung der mit dem Manipulatorarm 16 verbundenen Positionierhilfseinheit 600 zum Zieloperationsgebiet 30 nach einer zweiten Vorgehensweise, bei der im Unterschied zur ersten Vorgehensweise die Mittelachse 614 des Strahlenbündels 612 in einem ersten Schritt derart ausgerichtet wird, dass sie durch das Zieloperationsgebiet 30 verläuft und der Bedienperson ein entsprechendes optisches und/oder akustisches Signal ausgegeben wird. Die Ausrichtung der Mittelachse 614 zum Zieloperationsgebiet 30 kann dabei so erfolgen, wie beim Schritt 2 der ersten Vorgehensweise in Verbindung mit Figur 12 erläutert worden ist. Somit kann die Bedienperson auch hier optisch und/oder akustisch über den Abstand und/oder die Annährung der Mittelachse 614 zum Zieloperationsgebiet 30 informiert werden. Verläuft die Mittelachse 614 durch das Zieloperationsgebiet 30, wie dies für die Positionierhilfseinheit 600 in Figur 13 gezeigt ist, wird diese dann in Richtung des Pfeils A3 verschwenkt, bis die Mittelachse 614' des von der Positionierhilfseinheit 600 emittierten Strahlenbündels auf die Instrumentenöffnung des Trokars 800 trifft.

Anders als in Verbindung mit Figur 12 und 13 beschrieben, kann die Positionierhilfseinheit auch nur ein optisches und/oder akustisches Signal ausgeben oder beispielsweise über eine Pulsfolge des akustischen Signals und/oder des optischen Signals einer Bedienperson über die Pulsweite und/oder die Pulsdauer eine Information zu geben, wie groß der Betrag des orthogonalen Abstandsvektors V zum Zieloperationsgebiet 30 und/oder zum Mittelpunkt 31 des Zieloperationsgebiets 30 ist.

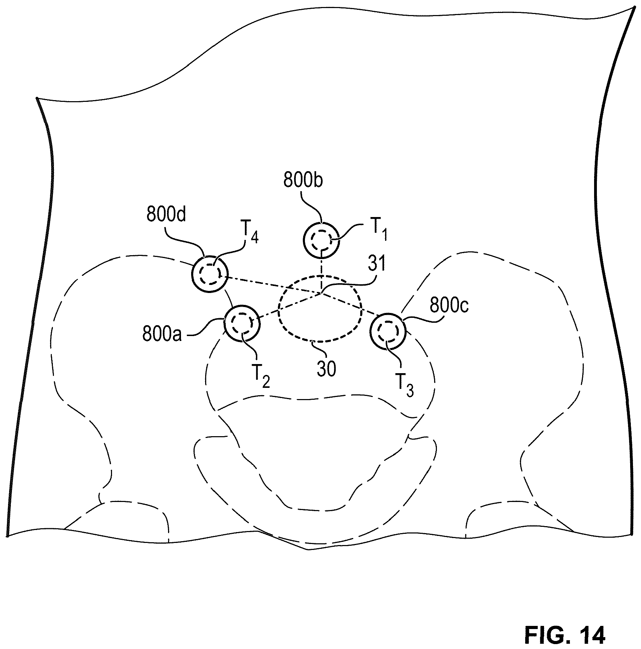

Figur 14 zeigt einen Ausschnitt des Körpers des Patienten 18 mit vier Körperöffnungen T1 bis T4, in die jeweils ein Trokar 800a bis 800d eingeführt ist. Durch den an der Eintrittsstelle T1 eingeführten Trokar 800b wird ein mit der Koppeleinheit 100b des Manipulatorarms 16b verbundenes Stabendoskop der Instrumenteneinheit 300b in den Körper des Patienten 18 eigeführt. Durch den an der Position T2 in den Körper des Patienten 18 eingeführten Trokar 800a wird ein chirurgisches Instrument 500a der mit der Koppeleinheit 100a des Manipulatorarms 16a verbundenen Instrumenteneinheit 300a in den Körper des Patienten 18 eingeführt. Durch einen an der Position T3 eingeführten Trokar 800c wird ein chirurgisches Instrument 500c der mit der Koppeleinheit 100c des Manipulatorarms 16c verbunden Instrumenteneinheit 300c in den Körper des Patienten 18 eingeführt. Durch den an der Position T4 in den Körper des Patienten 18 eingeführten Trokar 800d wird ein chirurgisches Instrument 500d der mit der Koppeleinheit 100d des Manipulatorarms 16d verbundenen Instrumenteneinheit 300d in den Körper des Patienten 18 eingeführt.

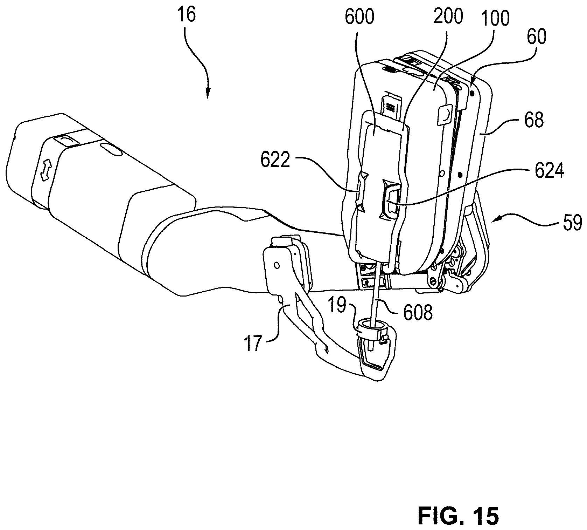

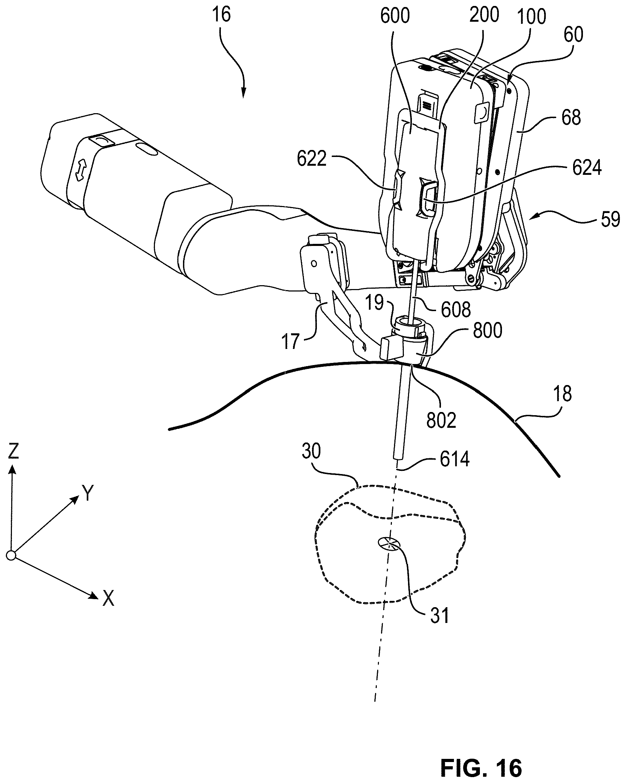

Figur 15 zeigt eine Anordnung mit dem distalen Ende des Manipulatorarms 16 mit der Koppeleinheit 100 und der mit der Koppeleinheit 100 verbundenen Positionierhilfseinheit 600 mit eingefahrener Teleskopanordnung 60 des Manipulatorarms16 gemäß einer zweiten Ausführungsform. In Figur 16 ist die Anordnung nach Figur 15 gezeigt, wobei die Trokar-Halterung 17 des Manipulatorarms 16 mit einem in den Patienten 18 eingeführten Trokar 800 verbunden ist. Die Anordnung der zweiten Ausführungsform gemäß den Figuren 15 und 16 unterscheidet sich von der in den Figuren 7 bis 14 gezeigten und erläuterten ersten Ausführungsform lediglich dadurch, dass eine Trokar-Halterung 17 fest mit dem Manipulatorarm 16 verbunden ist. Die Trokar-Halterung 17 hat ein Verbindungselement 19 zum Verbinden der Trokar-Halterung 17 mit dem Trokar 800. Die Positionierung und Ausrichtung des Manipulatorarms 16 mit Hilfe der Positionierhilfseinheit 600 erfolgt in gleicher Weise wie in Verbindung mit der ersten Ausführungsform beschrieben. Elemente mit gleicher Funktion oder gleichem Aufbau haben dieselben Bezugszeichen.

Nachdem die Ausrichtung der Positionierhilfseinheit 600 derart erfolgt ist, dass die Spitze des Schafts 608 der Positionierhilfseinheit 600 mit einer Länge im Bereich zwischen 1 cm und 6 cm, insbesondere mit einer Länge zwischen 2 cm und 4 cm, in den Trokar 800 eingeführt ist, ist das Verbindungselement 19 der Trokar-Halterung 17 automatisch so vorpositioniert, dass der Trokar 800 drehbeweglich mit dem Verbindungselement 19 der Trokar-Halterung 17 verbunden werden kann. Eine separate Ausrichtung der Verbindungseinheit 19 ist somit nicht erforderlich.

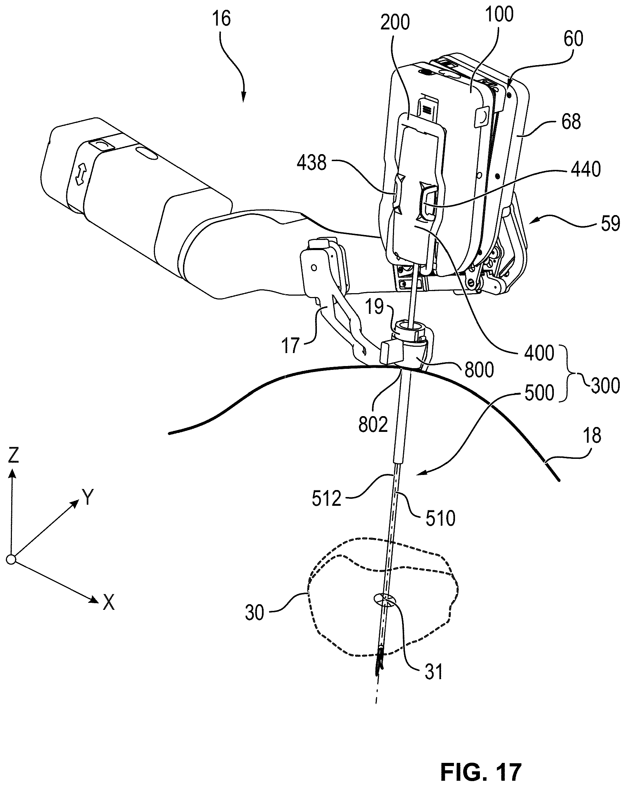

Figur 17 zeigt die Anordnung nach Figur 16, wobei bei gleicher Ausrichtung des Manipulatorarms 16, der Koppeleinheit 100 und der Trokar-Halterung 17 mit dem Verbindungselement 19 sowie dem Trokar 800 anstelle der Positionierhilfseinheit 600 die Instrumenteneinheit 300 mit der Koppeleinheit 100 des Manipulatorarms 16 verbunden ist. Zum Austausch der Positionierhilfseinheit 600 gegen die Instrumenteneinheit 300 ist die Teleskopanordnung 60 von der in den Figuren 15 bis 17 gezeigten eingefahrenen Position in ihre in Figur 9 gezeigte ausgefahrene Position bewegt worden, ohne dass die Ausrichtung des Manipulatorarms 16 der Koppeleinheit 100, der Trokar-Halterung 17 mit dem Verbindungselement 19 sowie dem Trokar 800 relativ zur Teleskopanordnung 60 geändert worden ist. Nach dem Austausch ist das chirurgische Instrument 500 der Instrumenteneinheit 300 in der in Figur 9 gezeigten Position angeordnet und kann durch Einfahren und Verschwenken der Teleskopanordnung 60 entlang der Längsachse 510 des Instrumentenschafts 512 bewegt werden, bis der Endeffektor 514 des chirurgischen Instruments 500 das Zieloperationsgebiet 30 erreicht oder in Richtung der Längsachse 510 des chirurgischen Instruments 500 über das Zieloperationsgebiet 30 hinausragt, wie dies in Figur 17 zu sehen ist.

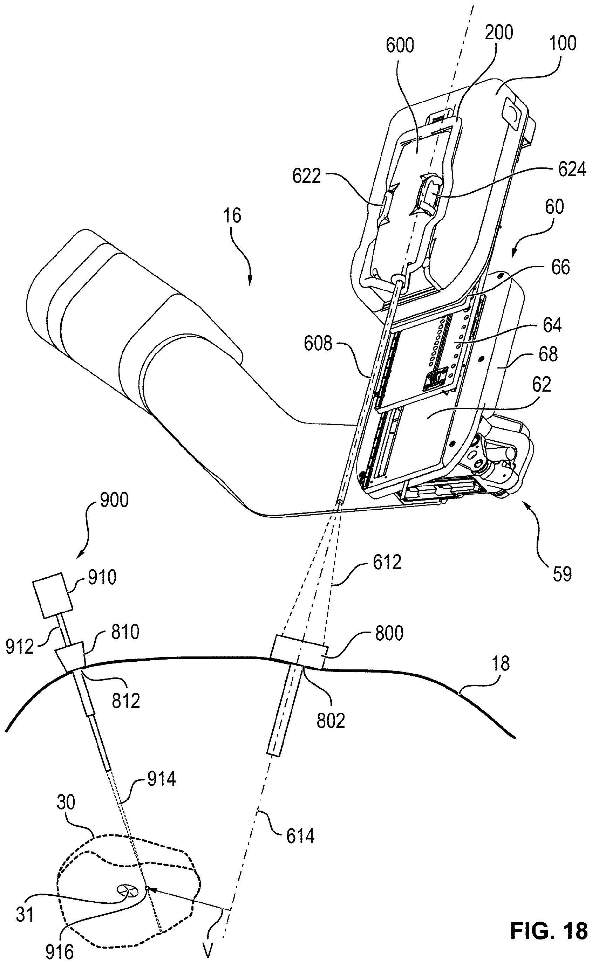

Figur 18 zeigt eine Anordnung mit einem Abschnitt eines Manipulatorarms 16 mit einer Koppeleinheit 100 und einem Endoskop 900 gemäß einer dritten Ausführungsform. Die Teleskopanordnung 60 des Manipulatorarms 16 ist in einem ausgefahrenen Zustand gezeigt. Ferner ist eine Positionierhilfseinheit 600 mit der Koppeleinheit 100 gekoppelt. Elemente mit demselben Aufbau oder gleicher Funktion haben dieselben Bezugszeichen.

Das Endoskop 900 ist ein Stabendoskop. Zumindest ein Teil der Abbildungsoptik des Stabendoskops 900 befindet sich in einem Stab 912, dessen proximales Ende über einen Trokar 810 über eine zweite Körperöffnung 812 in den Körper des Patienten 18 eingeführt ist. Mit Hilfe des Stabendoskops 900 werden Bilder zumindest eines Teils des Zieloperationsgebiets 30 aufgenommen. Das Stabendoskop 900 hat ein Kopfteil 910, über das das Stabendoskop 900 mit der Bedienkonsole 42 und/oder der Anzeigeeinheit 44 des Systems 10 zur robotergestützten Chirurgie verbunden ist. Dadurch kann einer Bedienperson, insbesondere einem Chirurgen das mit Hilfe des Endoskops 900 aufgenommene Bild an der Bedienkonsole 42 bzw. der Anzeigeeinheit 44 angezeigt werden.

Über das Kopfteil 910 kann das Stabendoskop 900 auch mit einer weiteren Koppeleinheit 100 insbesondere eines weiteren Manipulatorarms 16 gekoppelt sein. Durch Strichlinien sind die äußeren Abbildungslinien des Strahlengangs 914 der Abbildungsoptik des Stabendoskops 900 schematisch dargestellt. Die optische Achse der Abbildungsoptik des Stabendoskops 900, d. h. die optische Achse des Stabendoskops 900, liegt in der Mitte des Strahlengangs 914. Der Brennpunkt der Abbildungsoptik des Stabendoskops 900 ist mit 916 bezeichnet.

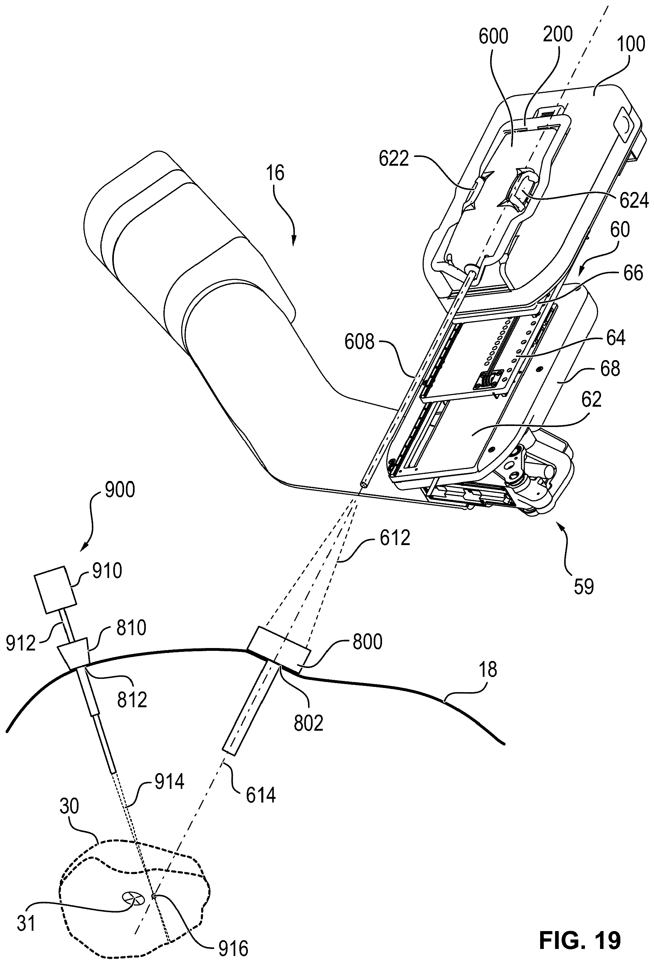

Im vorliegenden Ausführungsbeispiel wird der orthogonale Abstandsvektor V zwischen der Mittelachse 614 des Strahlengangs 612 des von der Positionierhilfseinheit 600 emittierten Lichts und dem Brennpunkt 916 der Abbildungsoptik des Stabendoskops 900 ermittelt. Der Brennpunkt 916 dient somit als Zielpunkt. Der ermittelte orthogonale Abstandsvektor V gibt den aktuellen Abstand zwischen der Mittelachse 614 des Strahlengangs 612 zum Brennpunkt 916 an. Die Auswertung der Position und die Ausrichtung des Manipulatorarms 16 mit der Positionierhilfseinheit 600 erfolgt dabei in gleicher Weise, wie dies in Verbindung mit den ersten beiden Ausführungsformen im Zusammenhang mit den Figuren 4 bis 17 bereits erläutert worden ist. Hierzu wird die Position der mit der Koppeleinheit 100 gekoppelten Positionierhilfseinheit 600 zusammen mit zumindest einem Teil der Segmente des Manipulatorarms 16 so verändert, dass die Mittelachse 614 des Strahlenbündels 612 sich dem als Zielpunkt dienenden Brennpunkt 916 bis zu einem voreingestellten ersten und/oder zweiten Abstandswert angenähert hat, oder wie in Figur 19 gezeigt, die Mittelachse 614 durch den Brennpunkt 916 verläuft. Hierzu ist in Figur 19 die Teleskopanordnung 60 des Manipulatorarms gegenüber der in Figur 18 gezeigten Position mit Hilfe des Koppelgetriebes 59 und/oder durch weitere Segmente des Manipulatorarms 16 verschwenkt worden.

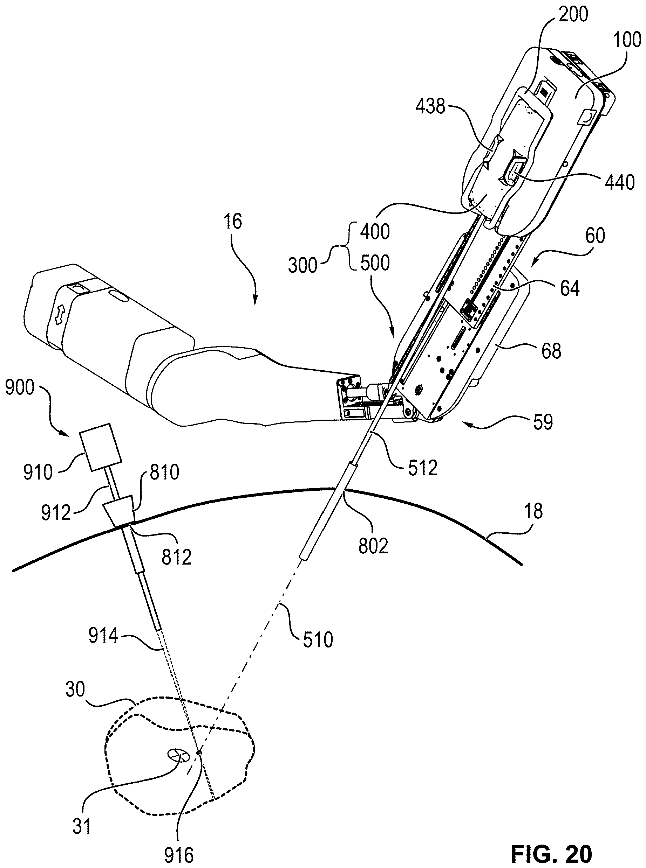

In Figur 20 ist die Anordnung nach Figur 19 gezeigt, wobei die Positionierhilfseinheit 600 gegen eine Instrumenteneinheit 300 mit einem chirurgischen Instrument 500 ausgetauscht worden ist ohne die Position der Koppeleinheit 100 des Manipulatorarms 16 zu ändern. Die Teleskopanordnung 60 befindet sich dabei in dem ausgefahrenen Zustand. Der Abstand zwischen der Positionierhilfseinheit 600 und dem Trokar 800 ist dabei, wie in Verbindung mit den ersten beiden Ausführungsbeispielen beschrieben, zuvor so eingestellt worden, dass nach dem Austausch der Positionierhilfseinheit 600 gegen die Instrumenteneinheit 300 das proximale Ende des Instrumentenschafts 512 des chirurgischen Instruments 500 der Instrumenteneinheit 300 in die Instrumentenöffnung des Trokars 800 eingeführt ist. Vorzugsweise ragt das Ende des Instrumentenschafts 512 mit seinem Endeffektor 514 mit einer Länge im Bereich von 2 cm bis 6 cm, insbesondere mit 4 cm in die Instrumentenöffnung des Trokars 800, so dass sich vorzugsweise der Endeffektor 514 in der Instrumentenöffnung des Trokars 800 befindet, wie dies in Figur 20 gezeigt ist.

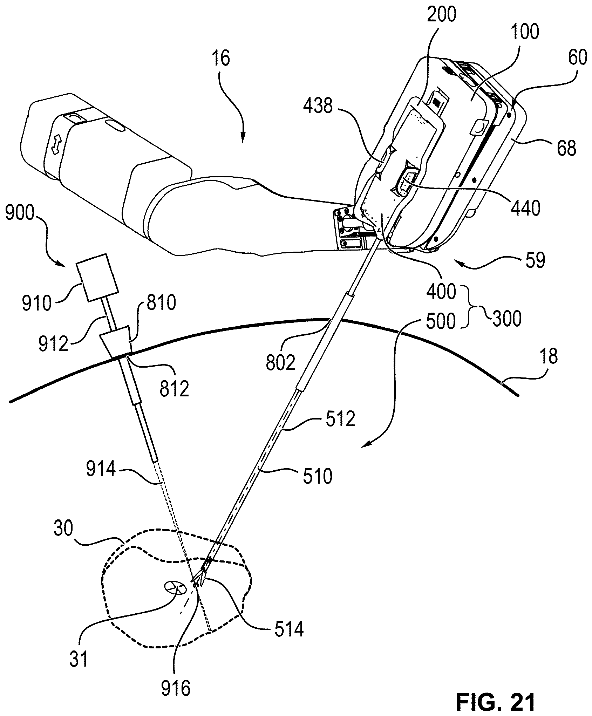

In Figur 21 ist die Anordnung nach Figur 20 mit eingefahrener Teleskopanordnung 60 gezeigt. Durch das Einfahren der Teleskopanordnung 60 ist der Endeffektor 514 bis in den Sichtbereich des Stabendoskops 900 und in den Zieloperationsbereich 30 bewegt worden, und kann dort unter Sichtkontrolle betätigt und positioniert werden. Durch die beschriebene Vorgehensweise ist es somit möglich, die Position, d. h. die Ausrichtung und die Lage, eines einzusetzenden chirurgischen Instruments 500 bereits so voreinzustellen, dass es zum Zieloperationsgebiet 30 geführt wird und dort sicher in den Blickbereich (field of view) des Stabendoskops 900 gebracht wird. Der Zielpunkt oder das Zielgebiet können durch Koordinaten $x_Z$, $y_Z$, $z_Z$ des Koordinatensystems X, Y, Z der Vorrichtung oder durch Koordinaten $x'_Z$, $y'_Z$, $z'_Z$ des Patientenkoordinatensystems X', Y', Z' festgelegt sein und erforderlichenfalls in Koordinaten des jeweils anderen Koordinatensystems umgerechnet werden.

Auch bei der ersten und bei der zweiten Ausführungsform nach den Figuren 4 bis 17 kann anstatt des Zieloperationsgebiets 30 ein Zielpunkt, wie z.B. der Mittelpunkt 31 des festgelegten Zieloperationsgebiets 30 oder ein von der Lage eines anderen chirurgischen Instruments 900 abhängiger Zielpunkt festgelegt sein. Auch kann anstatt des Endoskops 900 bei der dritten Ausführungsform ein anderes bildgebendes System zum Erfassen von Bildern zumindest eines Ausschnitts eines Zieloperationsgebiets 30 eingesetzt werden. Das Zielgebiet ist dann von der Lage des anderen bildgebenden Systems abhängig. Der Zielpunkt ist insbesondere ein Punkt im Schärfentiefebereich der Abbildungsoptik des Endoskops bzw. des bildgebenden Systems, wie beispielsweise der Brennpunkt der Abbildungsoptik oder ein Punkt zwischen dem Brennpunkt und dem proximalen Ende des Endoskops 900. Das Zielgebiet kann alternativ oder zusätzlich durch einen Abstand um einen Punkt festgelegt sein.

Die Vorgehensweise zum Positionieren des Manipulatorarms 16 wurde bei den drei Ausführungsformen in Verbindung mit der Positionierhilfseinheit 600 beschrieben. Anstelle der Positionierhilfseinheit 600 kann auch die Positionierhilfseinheit 700 eingesetzt werden, wobei die Positionierung des Manipulatorarms 16 und der Koppeleinheit 100 in gleicher Weise erfolgt, wie in Verbindung mit der Positionierhilfseinheit 600 beschrieben.

**Bezugszeichenliste**

| | |
|---|---|
| 10 | System |
| 12 | Manipulator |
| 14 | Stativ |
| 16, 16a bis 16d | Manipulatorarm |
| 17 | Trokar-Halterung |
| 18 | Patient |
| 19 | Verbindungselement |
| 20 | Stativkopf |
| 24 | Stativfuß |
| 28 | Stativarm |
| 30 | Zieloperationsgebiet |
| 31 | Mittelpunkt des Zieloperationsgebiet |
| 32 | Operationstischsäule |
| 34 | Operationstisch |
| 36 | Steuereinheit des Operationstisches |
| 38 | Patientenlagerfläche |
| 40 | zentrale Steuereinheit der Vorrichtung |
| 41 | Ausgabeeinheit |
| 42 | Bedienkonsole |
| 44 | Anzeigeeinheit |
| 46 | Steuereinheit des Manipulators |

| 47 | Ausgabeeinheit |
|---|---|
| 59 | Koppelgetriebe |
| 60 | Teleskopanordnung |
| 62, 64, 66 | Abschnitte der Teleskopanordnung |
| 68 | Antriebseinheit |
| 100, 100a bis 100d | Koppeleinheit |
| 102 | erste Übertragungsmittel |
| 104 | elektrisches Übertragungselement |
| 106, 108 | elektrischer Kontakt |
| 109 | optisches Übertragungselement |
| 110 | erstes translatorisches Antriebselement |
| 112 | zweites translatorisches Antriebselement |
| 114 | erstes rotatorisches Antriebselement |
| 116 | zweites rotatorisches Antriebselement |
| 120 | Koppelsensor |
| 121 | RFID-Lese- und Schreibeinheit |
| 122, 124 | Führungsnut |
| 123, 125 | vorderes Ende der Führungsnut |
| 126 | Rastnase |
| 128 | Rastelement |
| 134 | Entriegelungstaste |
| 200 | Sterilschleuse |
| 201 | Sterilfolie |
| 202 | Anschlussrand |
| 204, 206 | Führungsstift |
| 208, 210 | Schleusenklappe |
| 300, 300a bis 300d | Instrumenteneinheit |
| 400, 400a bis 400d | Sterileinheit |
| 438, 440 | Rast- und Betätigungselement |
| 494 | RFID-Transponder |
| 500, 500a bis 500d | chirurgisches Instrument |
| 510 | Längsachse |
| 512 | Instrumentenschaft |
| 514 | Endeffektor |
| 600, 700 | Positionierhilfseinheit |
| 602,604 | Sterilklappe |
| 606 | Gehäuse |
| 608 | Schaft |
| 610 | Lichtquelle |
| 611 | strahlformendes optisches Element |
| 612 | Strahlenbündel |
| 614 | Mittelachse |
| 616, 618 | elektrischer Kontakt |
| 620 | RFID-Transponder |
| 622, 624 | Rast- und Betätigungselement |
| 626 | Ausgabeeinheit |
| 702 | Energiequelle |
| 704 | elektronische Schaltung |
| 706 | Ausgabeeinheit |
| 800 | Trokar |
| 802 | Körperöffnung |
| 810 | Trokar |
| 812 | Körperöffnung |
| 900 | Stabendoskop |
| 910 | Kopfteil |
| 912 | Stab |
| 914 | Strahlengang |
| 916 | Brennpunkt |

| A1 bis A3 | Bewegungsrichtung |
| P1 bis P3 | Position |
| T1 bis T4 | geplante Körperöffnung |
| V, V' | Abstandsvektor |
| xz, yz, zz | Koordinaten des Zielgebiets im Koordinatensystem der Vorrichtung |
| X, Y, Z | Koordinatensystem der Vorrichtung |
| X', Y', Z' | Patientenkoordinatensystem |

**Patentansprüche**

1.  Vorrichtung zur robotergestützten Chirurgie,
    mit einer Instrumenteneinheit (300), die ein chirurgisches Instrument (500) mit einem Instrumentenschaft (512) hat, dessen proximales, dem Patienten zugewandtes Ende durch eine Körperöffnung (802) eines Patienten (18) zu einem durch Koordinaten ($x_z$, $y_z$, $z_z$) eines Koordinatensystems (X, Y, Z) der Vorrichtung festgelegten Zielgebiet (30) führbar ist,
    mit einer Positionierhilfseinheit (600, 700), die Licht als Strahlenbündel (612) emittiert,
    mit mindestens einer Koppeleinheit (100) eines Manipulatorarms (16), mit der wahlweise die Positionierhilfseinheit (600, 700) oder die Instrumenteneinheit (300) verbindbar ist,
    wobei bei einer Verbindung der Positionierhilfseinheit (600, 700) mit der Koppeleinheit (100) die Lage der Mittelachse (614) des von der Positionierhilfseinheit (600, 700) emittierten Strahlenbündels (612) mit der Lage der Längsachse (510) des Instrumentenschafts (512) der anstatt der Positionierhilfseinheit (600, 700) mit der Koppeleinheit (100) verbundenen Instrumenteneinheit (300) übereinstimmt,
    mit einer Steuereinheit (40, 46), **dadurch gekennzeichnet, dass** die Steuereinheit (40, 46)

    - bei einer Verbindung der Positionierhilfseinheit (600, 700) mit der Koppeleinheit (100) den zur Mittelachse (614) orthogonalen Abstandsvektor (V) zwischen der Mittelachse (614) und dem durch die Koordinaten ($x_z$, $y_z$, $z_z$) festgelegten Zielgebiet (30) ermittelt,
    - eine erste Steuerinformation erzeugt, wenn der Betrag des ermittelten Abstandsvektors (V) einen ersten voreingestellten Wert hat und/oder unterschreitet, und

    mit einer Ausgabeeinheit (41, 47, 626, 706), die abhängig von der ersten Steuerinformation ein Signal ausgibt.

2.  Vorrichtung nach Anspruch 1, **dadurch gekennzeichnet, dass** die Steuereinheit (40, 46) zumindest eine zweite Steuerinformation erzeugt, wenn der Betrag des ermittelten Abstandsvektors (V) einen zweiten voreingestellten Wert hat und/oder unterschreitet, und dass die Ausgabeeinheit (41, 47, 626, 706) abhängig von der zweiten Steuerinformation ein Signal ausgibt.

3.  Vorrichtung nach einem der vorhergehenden Ansprüche, **dadurch gekennzeichnet, dass** die Ausgabeeinheit (41, 47) der Vorrichtung und/oder die Ausgabeeinheit (626, 706) der Positionierhilfseinheit (600, 700) auf Grund der ersten Steuerinformation ein erstes akustisches und/oder ein erstes optisches Signal ausgibt, und/oder dass die Ausgabeeinheit (41, 47, 626, 706) auf Grund der zweiten Steuerinformation nur ein zweites akustisches und/oder nur ein zweites optisches Signal ausgibt.

4.  Vorrichtung nach einem der vorhergehenden Ansprüche, **dadurch gekennzeichnet, dass** die Steuereinheit (40, 46) die erzeugte erste Steuerinformation und/oder die erzeugte zweite Steuerinformation zur Positionierhilfseinheit (600, 700) überträgt und dass die Positionierhilfseinheit (600, 700) die Ausgabeeinheit (626, 706) umfasst.

5.  Vorrichtung nach Anspruch 3 oder 4, **dadurch gekennzeichnet, dass** das erste akustische Signal ein an- und abschwellender Ton oder eine Tonfolge mit einer ersten Wiederholfrequenz ist und dass das zweite akustische Signal ein Dauerton ist.

6.  Vorrichtung nach einem der Ansprüche 3 bis 5, **dadurch gekennzeichnet, dass** das erste optische Signal ein blinkendes Lichtsignal mit einer ersten Blinkfrequenz ist und dass das zweite optische Signal ein Lichtsignal mit einer zweiten Blinkfrequenz ist, die auch null sein kann.

7.  Vorrichtung nach einem der vorhergehenden Ansprüche 3, 4 oder 6, **dadurch gekennzeichnet, dass** die Positionierhilfseinheit (600, 700) zum Erzeugen des ersten optischen Signals Licht mit einer ersten Wellenlänge und zum Erzeugen des zweiten optischen Signals Licht mit einer von der ersten Wellenlänge verschiedenen zweiten Wellenlänge emittiert.

8. Vorrichtung nach einem der vorherigen Ansprüche 3, 4, 6 oder 7, **dadurch gekennzeichnet, dass** zum Erzeugen des ersten und/oder zweiten optischen Signals mit Hilfe des Strahlenbündels (612) eine Abbildung eines Fadenkreuzes und/oder mindestens eines um die Mittelachse (614) konzentrisch angeordneten Kreises im Bereich einer geplanten oder vorhandenen Körperöffnung (802) des Patienten (18) erzeugt wird.

9. Vorrichtung nach einem der vorhergehenden Ansprüche, **dadurch gekennzeichnet, dass** die Positionierhilfseinheit (600, 700) eine Energiequelle (702) zum Versorgen einer Signalerzeugungseinheit (626,706) zum Erzeugen des ersten und/oder zweiten optischen und/oder akustischen Signals hat.

10. Vorrichtung nach einem der vorhergehenden Ansprüche, **dadurch gekennzeichnet, dass** die Positionierhilfseinheit eine elektronische Schaltung (704) mit einer Schnittstelle zu einer Steuereinheit (40, 46) der Vorrichtung zum Empfangen einer ersten Steuerinformation hat, die angibt, dass der Betrag eines ermittelten zur Mittelachse (614) orthogonalen Abstandsvektors (V) zwischen einem durch Koordinaten $(x_z, y_z, z_z)$ festgelegten Zielgebiet (30) und der Mittelachse (614) einen voreingestellten Wert hat oder unterschreitet.

11. Vorrichtung nach Anspruch 10, **dadurch gekennzeichnet, dass** die Schnittstelle der elektronischen Schaltung (704) weiterhin zum Empfangen einer zweiten Steuerinformation dient, die angibt, dass der Betrag des ermittelten zur Mittelachse (614) orthogonalen Abstandsvektors (V) zwischen den Koordinaten $(x_z, y_z, z_z)$ des Zielgebiets (30) und der Mittelachse (614) einen zweiten voreingestellten Wert erreicht und/oder unterschreitet.

12. Verfahren zum Positionieren eines Manipulatorarms in einem Koordinatensystem (X, Y, Z) einer Vorrichtung zur robotergestützten Chirurgie vor einem chirurgischen Eingriff an einem Patienten,
bei dem die Koordinaten $(x_z, y_z, z_z)$ eines Zielgebietes (30) eines Patienten (18) ermittelt werden,
bei dem zum Positionieren eines Manipulatorarms (16) eine Positionierhilfseinheit (600, 700) anstelle einer Instrumenteneinheit (300) mit einer Koppeleinheit (100) des Manipulatorarms (16) verbunden wird,
bei dem Licht mit Hilfe der Positionierhilfseinheit (600, 700) als Strahlenbündel (612) emittiert wird, wobei die Lage der Mittelachse (614) des von der mit der Koppeleinheit (100) verbundenen Positionierhilfseinheit (600, 700) emittierten Strahlenbündels (612) mit der Lage der Längsachse (510) eines chirurgischen Instruments (500) der anstelle der Positionierhilfseinheit (600, 700) mit der Koppeleinheit (100) verbundenen Instrumenteneinheit (300) übereinstimmt,
bei dem bei einer Verbindung der Positionierhilfseinheit (600, 700) mit der Koppeleinheit (100) mit Hilfe einer Steuereinheit (40, 46) der zur Mittelachse (614) orthogonale Abstandsvektor (V) zwischen der Mittelachse (614) und dem durch die Koordinaten $(x_z, y_z, z_z)$ festgelegte Zielgebiet (30) ermittelt wird, und
bei dem mit Hilfe einer Ausgabeeinheit (41, 47, 626, 706) ein erstes optisches und/oder akustisches Signal ausgegeben wird, wenn der Betrag des ermittelten Abstandsvektors (V) einen ersten voreingestellten Wert hat und/oder unterschreitet.

13. Verfahren nach Anspruch 12, **dadurch gekennzeichnet, dass** ein zweites optisches und/oder akustisches Signal ausgegeben wird, wenn der Betrag des ermittelten Abstandsvektors (V) einen zweiten voreingestellten Wert erreicht und/oder unterschreitet.

14. Verfahren nach Anspruch 12 oder 13, **dadurch gekennzeichnet, dass** der Manipulatorarm (16) so ausgerichtet wird, dass die Mittelachse (614) des Strahlenbündels (612) durch eine geplante operative Körperöffnung (802) eines Patienten (18) verläuft und das erste und/oder zweite optische und/oder akustische Signal ausgegeben wird.

15. Verfahren nach einem der vorhergehenden Ansprüche 12 bis 14, **dadurch gekennzeichnet, dass** der Manipulatorarm (16) in einem ersten Schritt so ausgerichtet wird, dass die Mittelachse (614) des Strahlenbündels (612) durch eine geplante operative Körperöffnung (802) des Patienten (18) verläuft, und dass der Manipulatorarm (16) in einem zweiten Schritt so bewegt wird, bis der mit Hilfe der Steuereinheit (40, 46) ermittelte Betrag des Abstandsvektors (V) zwischen der durch die geplante operative Körperöffnung (802) des Patienten (18) verlaufende Mittelachse (614) und dem durch die Koordinaten $(x_z, y_z, z_z)$ festgelegten Zielgebiet (30) den ersten und/oder zweiten Wert erreicht oder unterschreitet,
wobei der Manipulatorarm (16) automatisch durch die Vorrichtung selbst und/oder manuell bewegt wird.

16. Verfahren nach einem der vorhergehenden Ansprüche 12 bis 15, **dadurch gekennzeichnet, dass** der Manipulatorarm (16) in einem ersten Schritt so ausgerichtet wird, dass der mit Hilfe der Steuereinheit (40, 46) ermittelte Betrag des Abstandsvektors (V) zwischen der Mittelachse (614) und dem durch die Koordinaten ($x_z$, $y_z$, $z_z$) festgelegten Zielgebiet (30) den ersten und/oder zweiten Wert erreicht oder unterschreitet, und dass der Manipulatorarm (16) in einem zweiten Schritt so ausgerichtet wird, dass die Mittelachse (614) des Strahlenbündels (612) durch die vorhandene oder geplante operative Körperöffnung (802) des Patienten (18) verläuft, wobei der Manipulatorarm (16) automatisch durch die Vorrichtung selbst und/oder manuell bewegt wird.

17. Vorrichtung oder Verfahren nach einem der vorhergehenden Ansprüche, **dadurch gekennzeichnet, dass** das Zielgebiet durch ein Zieloperationsgebiet (30), durch einen Mittelpunkt (31) eines Zieloperationsgebietes (30) oder durch die Lage eines anderen chirurgischen Instruments (300, 900) festgelegt ist.

18. Vorrichtung oder Verfahren nach einem der vorhergehenden Ansprüche, **dadurch gekennzeichnet, dass** das Zielgebiet durch die Lage eines zumindest teilweise in den Körper des Patienten (18) eingeführten Endoskops (900) oder eines anderen bildgebenden Systems zum Erfassen von Bildern zumindest eines Ausschnitts eines Zieloperationsgebiets (30) festgelegt ist, vorzugsweise durch die optische Achse und/oder den Brennpunkt (916) der Abbildungsoptik des Endoskops bzw. des bildgebenden Systems.

**Claims**

1. An apparatus for robot-assisted surgery, comprising
an instrument unit (300) having a surgical instrument (500) with an instrument shaft (512), the proximal end of which, facing the patient, is passable through a body orifice (802) of a patient (18) to a target area (30) defined by coordinates ($x_z$, $y_z$, $z_z$) of a coordinate system (X, Y, Z) of the apparatus (30),
a positioning device (600, 700) emitting light as a beam of rays (612),
at least one coupling unit (100) of a manipulator arm (16), to which optionally the positioning device (600, 700) or the instrument unit (300) is connectable,
wherein, when connecting the positioning device (600, 700) to the coupling unit (100), the position of the central axis (614) of the beam of rays (612) emitted by the positioning device (600, 700) corresponds with the position of the longitudinal axis (510) of the instrument shaft (512) of the instrument unit (300) connected to the coupling unit (100) instead of the positioning device (600, 700),
a control unit (40, 46),
**characterized in that** the control unit (40, 46)

   - when the positioning device (600, 700) is connected to the coupling unit (100), determines the distance vector (V), which is orthogonal to the central axis (614), between the central axis (614) and the target area (30) defined by the coordinates ($x_z$, $y_z$, $z_z$),
   - generates a first control information when the amount of the determined distance vector (V) has and/or falls below a first preset value, and

comprising an output unit (41, 47, 626, 706) which outputs a signal dependent on the first control information.

2. The apparatus according to claim 1, **characterized in that** the control unit (40, 46) generates at least a second control information, when the amount of the determined distance vector (V) has and/or falls below a second preset value, and that the output unit (41, 47, 626, 706) outputs a signal dependent on the second control information.

3. The apparatus according to one of the preceding claims, **characterized in that** the output unit (41, 47) of the apparatus and/or the output unit (626, 706) of the positioning device (600, 700) outputs a first acoustic and/or a first optical signal on the basis of the first control information, and/or that the output unit (41, 47, 626, 706) outputs only a second acoustic and/or only a second optical signal on the basis of the second control information.

4. The apparatus according to one of the preceding claims, **characterized in that** the control unit (40, 46) transmits the generated first control information and/or the generated second control information to the positioning device (600, 700) and that the positioning device (600, 700) comprises the output unit (626, 706).

5. The apparatus according to claim 3 or 4, **characterized in that** the first acoustic signal is a swelling and falling tone or a tone sequence with a first repetition rate and that the second acoustic signal is a continuous tone.

6. The apparatus according to one of the claims 3 to 5, **characterized in that** the first optical signal is a blinking light signal with a first blinking rate and that the second optical signal is a light signal with a second blinking rate, which may also be zero.

7. The apparatus according to one of the preceding claims 3, 4 or 6, **characterized in that** the positioning device (600, 700) for generating the first optical signal emits light with a first wavelength and for generating the second optical signal emits light with a second wavelength different from the first wavelength.

8. The apparatus according to one of the preceding claims 3, 4, 6 or 7, **characterized in that** for generating the first and/or the second optical signal by means of the beam of rays (612) an image of a cross hair and/or at least one circle concentrically arranged around the central axis (614) is generated in the area of a planned or existing body orifice (802) of the patient (18).

9. The apparatus according to one of the preceding claims, **characterized in that** the positioning device (600, 700) has an energy source (702) for supplying a signal generating unit (626, 706) for generating the first and/or second optical and/or acoustic signal.

10. The apparatus according to one of the preceding claims, **characterized in that** the positioning device has an electronic circuit (704) having an interface to a control unit (40, 46) of the apparatus for receiving a first control information which indicates that the amount of a determined distance vector (V), which is orthogonal to the central axis (614), between a target area (30) defined by coordinates $(x_z, y_z, z_z)$ and the central axis (614) has or falls below a preset value.

11. The apparatus according to claim 10, **characterized in that** the interface of the electronic circuit (704) further serves to receive a second control information which indicates that the amount of the determined distance vector (V), which is orthogonal to the central axis (614), between the coordinates $(x_z, y_z, z_z)$ of the target area (30) and the central axis (614) reaches and/or falls below a second preset value.

12. A method for positioning a manipulator arm in a coordinate system (X, Y, Z) of an apparatus for robot-assisted surgery prior to a surgery on a patient,
in which the coordinates $(x_z, y_z, z_z)$ of a target area (30) of a patient (18) are determined,
in which for positioning a manipulator arm (16) a positioning device (600, 700) is connected to a coupling unit (100) of the manipulator arm (16) instead of an instrument unit (300),
in which light is emitted by means of the positioning device (600, 700) as a beam of rays (612), wherein the position of the central axis (614) of the beam of rays (612) emitted by the positioning device (600, 700) connected to the coupling unit (100) corresponds with the position of the longitudinal axis (510) of a surgical instrument (500) of the instrument unit (300) connected to the coupling unit (100) instead of the positioning device (600, 700),
in which, when the positioning device (600, 700) is connected to the coupling unit (100), the distance vector (V), which is orthogonal to the central axis (614), between the central axis (614) and the target area (30) defined by the coordinates $(x_z, y_z, z_z)$ is determined by means of a control unit (40, 46), and
in which a first optical and/or acoustic signal is output by means of an output unit (41, 47, 626, 706) when the amount of the determined distance vector (V) has and/or falls below a first preset value.

13. The method according to claim 12, **characterized in that** a second optical and/or acoustic signal is output when the amount of the determined distance vector (V) has reached and/or falls below a second preset value.

14. The method according to claim 12 or 13, **characterized in that** the manipulator arm (16) is oriented such that the central axis (614) of the beam of rays (612) runs through a planned operative body orifice (802) of a patient (18) and the first and/or second optical and/or acoustic signal is output.

15. The method according to one of the preceding claims 12 to 14, **characterized in that** the manipulator arm (16) is oriented in a first step such that the central axis (614) of the beam of rays (612) runs through a planned operative body orifice (802) of the patient (18), and that the manipulator arm (16) is moved in a second step until the amount of the distance vector (V), determined by means of the control unit (40, 46), between the central axis (614) running through the planned operative body orifice (802) of the patient (18) and the target area (30) defined by the coordinates $(x_z, y_z, z_z)$ reaches or falls below the first and/or second value,
wherein the manipulator arm (16) is moved automatically by the apparatus itself and/or manually.

**16.** The method according to one of the preceding claims 12 to 15, **characterized in that** the manipulator arm (16) is oriented in a first step such that the amount of the distance vector (V), determined by means of the control unit (40, 46), between the central axis (614) and the target area (30) defined by the coordinates ($x_z$, $y_z$, $z_z$) reaches and/or falls below the first and/or second value,

and that the manipulator arm (16) is oriented in a second step such that the central axis (614) of the beam of rays (612) runs through the existing or planned operative body orifice (802) of the patient (18),

wherein the manipulator arm (16) is moved automatically by the apparatus itself and/or manually.

**17.** The apparatus or the method according to one of the preceding claims, **characterized in that** the target area is defined by a target surgical area (30), by a center (31) of a target surgical area (30) or by the position of another surgical instrument (300, 900).

**18.** The apparatus or method according to one of the preceding claims, **characterized in that** the target area is defined by the position of an endoscope (900) at least partly inserted into the body of the patient (18) or another imaging system for capturing images of at least a detail of a target surgical area (30), preferably by the optical axis and/or the focal point (916) of the imaging optics of the endoscope or the imaging system.

## Revendications

**1.** Dispositif de chirurgie assisté par un robot,

comprenant une unité instrument (300) qui présente un instrument chirurgical (500) pourvu d'une tige d'instrument (512) dont l'extrémité proximale, tournée vers le patient, peut être guidée à travers un orifice corporel (802) d'un patient (18) en direction d'une zone cible (30) définie par les coordonnées ($x_z$, $y_z$, $z_z$) d'un système de coordonnées (X, Y, Z) du dispositif,

comprenant une unité d'aide au positionnement (600, 700), qui émet une lumière sous la forme d'un faisceau lumineux (612),

comprenant au moins une unité d'accouplement (100) d'un bras de manipulateur (16), à laquelle sélectivement l'unité d'aide au positionnement (600, 700) ou l'unité instrument (300) peut être reliée,

la position de l'axe médian (614) du faisceau lumineux (612) émis par l'unité d'aide au positionnement (600, 700), lorsque l'unité d'aide au positionnement (600, 700) est reliée à l'unité d'accouplement (100), coïncidant avec la position de l'axe longitudinal (510) de la tige d'instrument (512) de l'unité d'instrument (300) reliée à l'unité d'accouplement (100) en lieu et place de l'unité d'aide au positionnement (600, 700),

comprenant une unité de commande (40, 46),

**caractérisé en ce que** l'unité de commande (40, 46)

- lorsque l'unité d'aide au positionnement (600, 700) est reliée à l'unité d'accouplement (100), détermine le vecteur de distance (V) perpendiculaire à l'axe médian (614) entre l'axe médian (614) et la zone cible (30) définie par les coordonnées ($x_z$, $y_z$, $z_z$),

- génère une première information de commande, lorsque la grandeur du vecteur de distance (V) déterminé présente une première valeur préréglée et/ou est inférieure à celle-ci, et

comprenant une unité de sortie (41, 47, 626, 706) qui délivre un signal en fonction de la première information de commande.

**2.** Dispositif selon la revendication 1, **caractérisé en ce que** l'unité de commande (40, 46) génère au moins une deuxième information de commande, lorsque la grandeur du vecteur de distance (V) déterminé présente une deuxième valeur préréglée et/ou est inférieure à celle-ci, et **en ce que** l'unité de sortie (41, 47, 626, 706) délivre un signal en fonction de la deuxième information de commande.

**3.** Dispositif selon l'une quelconque des revendications précédentes, **caractérisé en ce que** l'unité de sortie (41, 47) du dispositif et/ou l'unité de sortie (626, 706) de l'unité de positionnement (600, 700) délivre un premier signal acoustique et/ou un premier signal optique sur la base de la première information de commande, et/ou **en ce que** l'unité de sortie (41, 47, 626, 706) délivre un seul deuxième signal acoustique et/ou un seul deuxième signal optique sur la base de la deuxième information de commande.

**4.** Dispositif selon l'une quelconque des revendications précédentes, **caractérisé en ce que** l'unité de commande (40, 46) transmet la première information de commande générée et/ou la deuxième information de commande

# EP 3 103 409 B1

générée à l'unité d'aide au positionnement (600, 700) et **en ce que** l'unité d'aide au positionnement (600, 700) comprend l'unité de sortie (626, 706).

5. Dispositif selon la revendication 3 ou 4, **caractérisé en ce que** le premier signal acoustique est un son ou une succession de sons qui va en augmentant et en descendant à une première fréquence de répétition et **en ce que** le deuxième signal acoustique est un son continu.

6. Dispositif selon l'une quelconque des revendications 3 à 5, **caractérisé en ce que** le premier signal optique est un signal lumineux clignotant à une première fréquence de clignotement et **en ce que** le deuxième signal optique est un signal lumineux présentant une deuxième fréquence de clignotement qui peut également être nulle.

7. Dispositif selon l'une quelconque des revendications précédentes 3, 4 ou 6, **caractérisé en ce que** l'unité d'aide au positionnement (600, 700), pour la génération du premier signal optique, émet une lumière d'une première longueur d'onde, et, pour la génération du deuxième signal optique, émet une lumière d'une deuxième longueur d'onde différente de la première longueur d'onde.

8. Dispositif selon l'une quelconque des revendications 3, 4, 6 ou 7, **caractérisé en ce que**, pour la génération du premier et/ou du deuxième signal optique, une reproduction d'une ligne de mire et/ou d'au moins un cercle situé de manière concentrique autour de l'axe médian (614) est générée dans la zone d'un orifice corporel (802) planifié ou présent du patient (18) à l'aide du faisceau lumineux (612).

9. Dispositif selon l'une quelconque des revendications précédentes, **caractérisé en ce que** l'unité d'aide au positionnement (600, 700) présente une source d'énergie (702) servant à alimenter une unité de génération de signaux (626, 706) pour la génération du premier et/ou du deuxième signal optique et/ou acoustique.

10. Dispositif selon l'une quelconque des revendications précédentes, **caractérisé en ce que** l'unité d'aide au positionnement présente un circuit électronique (704) pourvu d'une interface avec une unité de commande (40, 46) du dispositif pour la réception d'une première information de commande, qui indique que la grandeur d'un vecteur de distance (V) déterminé perpendiculaire à l'axe médian (614) entre une zone cible (30) définie par les coordonnées $(x_z, y_z, z_z)$ et l'axe médian (614) présente une valeur préréglée ou est inférieure à celle-ci.

11. Dispositif selon la revendication 10, **caractérisé en ce que** l'interface du circuit électronique (704) sert en outre à recevoir une deuxième information de commande qui indique que la grandeur du vecteur de distance (V) déterminé perpendiculaire à l'axe médian (614) entre les coordonnées $(x_z, y_z, z_z)$ de la zone cible (30) et l'axe médian (614) atteint une deuxième valeur préréglée et/ou est inférieure à celle-ci.

12. Procédé de positionnement d'un bras de manipulateur dans un système de coordonnées (X, Y, Z) d'un dispositif pour la chirurgie assistée par un robot avant une intervention chirurgicale sur un patient,
selon lequel les coordonnées $(x_z, y_z, z_z)$ d'une zone cible (30) d'un patient (18) sont déterminées,
selon lequel, pour le positionnement d'un bras de manipulateur (16), une unité d'aide au positionnement (600, 700), en lieu et place d'une unité instrument (300), est reliée à une unité d'accouplement (100) du bras de manipulateur (16), selon lequel une lumière est émise sous la forme d'un faisceau lumineux (612) à l'aide de l'unité d'aide au positionnement (600, 700), la position de l'axe médian (614) du faisceau lumineux (612) émis par l'unité d'aide au positionnement (600, 700) reliée à l'unité d'accouplement (100) coïncidant avec la position de l'axe longitudinal (510) d'un instrument chirurgical (500) de l'unité instrument (300) reliée à l'unité d'accouplement (100) en lieu et place de l'unité d'aide au positionnement (600, 700),
selon lequel, lorsque l'unité d'aide au positionnement (600, 700) est reliée à l'unité d'accouplement (100), le vecteur de distance (V) perpendiculaire à l'axe médian (614) entre l'axe médian (614) et la zone cible (30) définie par les coordonnées $(x_z, y_z, z_z)$ est déterminé à l'aide d'une unité de commande (40, 46), et
selon lequel un premier signal optique et/ou acoustique est délivré à l'aide d'une unité de sortie (41, 47, 626, 706), lorsque la grandeur du vecteur de distance (V) déterminé présente une première valeur préréglée ou est inférieure à celle-ci.

13. Procédé selon la revendication 12, **caractérisé en ce qu'**un deuxième signal optique et/ou acoustique est délivré, lorsque la grandeur du vecteur de distance (V) déterminé atteint une deuxième valeur préréglée et/ou est inférieure à celle-ci.

14. Procédé selon la revendication 12 ou 13, **caractérisé en ce que** le bras de manipulateur (16) est orienté de sorte que l'axe médian (614) du faisceau lumineux (612) s'étend à travers un orifice corporel (802) opératoire planifié d'un patient (18) et le premier et/ou deuxième signal optique et/ou acoustique est délivré.

**15.** Procédé selon l'une quelconque des revendications 12 à 14, **caractérisé en ce que** le bras de manipulateur (16) est orienté au cours d'une première étape de sorte que l'axe médian (614) du faisceau lumineux (612) s'étend à travers un orifice corporel (802) opératoire planifié du patient (18), et **en ce que** le bras de manipulateur (16) est déplacé au cours d'une deuxième étape jusqu'à ce que la grandeur du vecteur de distance (V) déterminée à l'aide de l'unité de commande (40, 46), entre l'axe médian (614) s'étendant à travers l'orifice corporel (802) opératoire planifié du patient (18) et la zone cible (30) définie par les coordonnées ($x_z$, $y_z$, $z_z$), atteigne la première et/ou la deuxième valeur ou soit inférieure à celle-ci,
le bras de manipulateur (16) étant déplacé automatiquement par le dispositif lui-même et/ou manuellement.

**16.** Procédé selon l'une quelconque des revendications 12 à 15, **caractérisé en ce que** le bras de manipulateur (16) est orienté au cours d'une première étape de sorte que la grandeur du vecteur de distance (V) déterminée à l'aide de l'unité de commande (40, 46), entre l'axe médian (614) et la zone cible (30) définie par les coordonnées ($x_z$, $y_z$, $z_z$), atteint la première et/ou la deuxième valeur ou est inférieure à celle-ci,
et **en ce que** le bras de manipulateur (16), au cours d'une deuxième étape, est orienté de sorte que l'axe médian (614) du faisceau lumineux (612) s'étend à travers l'orifice corporel (802) opératoire présent ou planifié du patient (18) le bras de manipulateur (16) étant déplacé automatiquement par le dispositif lui-même et/ou manuellement.

**17.** Dispositif ou procédé selon l'une quelconque des revendications précédentes, **caractérisé en ce que** la zone cible est définie par une zone opératoire cible (30), par un centre (31) d'une zone opératoire cible (30) ou par la position d'un autre instrument chirurgical (300, 900).

**18.** Dispositif ou procédé selon l'une quelconque des revendications précédentes, **caractérisé en ce que** la zone cible est définie par la position d'un endoscope (900) introduit au moins en partie dans le corps du patient (18) ou d'un autre système d'imagerie pour l'acquisition d'images d'au moins une section d'une zone opératoire cible (30), de préférence par l'axe optique et/ou le foyer (916) de l'optique de reproduction de l'endoscope ou du système d'imagerie.

FIG. 1

**FIG. 2**

FIG. 3

EP 3 103 409 B1

FIG. 4

FIG. 5

FIG. 6

EP 3 103 409 B1

**FIG. 7**

**FIG. 8**

**FIG. 9**

**FIG. 10**

**FIG. 11**

FIG. 13

FIG. 12

FIG. 14

FIG. 15

**FIG. 16**

FIG. 17

**FIG. 18**

**FIG. 19**

**FIG. 20**

**FIG. 21**

**IN DER BESCHREIBUNG AUFGEFÜHRTE DOKUMENTE**

**In der Beschreibung aufgeführte Patentdokumente**

- DE 102014117407 A1
- DE 102014117408 A1
- US 20110152871 A1
- US 20140092587 A1
- US 20100076305 A1
- US 7666191 B1
- DE 10242953 A1